(19) Europäisches Patentamt
European Patent Office
Office européen des brevets

(11) EP 4 548 854 A1

(12) **EUROPEAN PATENT APPLICATION**
published in accordance with Art. 153(4) EPC

(43) Date of publication:
**07.05.2025 Bulletin 2025/19**

(21) Application number: **23831442.1**

(22) Date of filing: **27.06.2023**

(51) International Patent Classification (IPC):
***A61B 8/00*** $^{(2006.01)}$ ***H04R 17/00*** $^{(2006.01)}$

(52) Cooperative Patent Classification (CPC):
**A61B 8/4444; A61B 8/00; H04R 17/00**

(86) International application number:
**PCT/JP2023/023757**

(87) International publication number:
**WO 2024/005002 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.06.2022 JP 2022104704**

(71) Applicant: **FUJIFILM Corporation**
**Tokyo 106-8620 (JP)**

(72) Inventor: **NAKAI, Yoshihiro**
**Ashigarakami-gun, Kanagawa 258-8577 (JP)**

(74) Representative: **HGF**
**HGF Limited**
**1 City Walk**
**Leeds LS11 9DX (GB)**

(54) **ACOUSTIC WAVE PROBE, ACOUSTIC WAVE MEASUREMENT DEVICE, AND ULTRASONIC DIAGNOSIS DEVICE**

(57) Provided are an acoustic wave probe including a substrate, an interlayer on the substrate, and an acoustic lens layer on the interlayer, in which the interlayer is a layer containing a siloxane compound, the siloxane compound has a constitutional component derived from a hydrolyzed silicate compound, and the acoustic lens layer is a layer obtained by curing room temperature-curable silicone, in which the room temperature-curable silicone includes a constitutional unit having a phenyl group; an acoustic wave measurement apparatus; and an ultrasound diagnostic apparatus.

FIG. 1

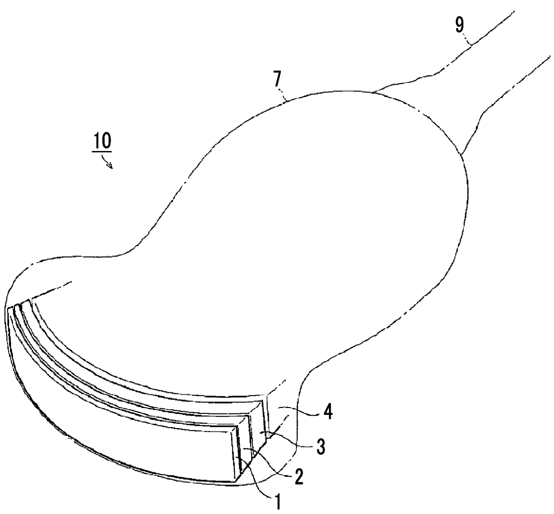


EP 4 548 854 A1

## Description

### BACKGROUND OF THE INVENTION

1. Field of the Invention

The present invention relates to an acoustic wave probe, an acoustic wave measurement apparatus, and an ultrasound diagnostic apparatus.

2. Description of the Related Art

In an acoustic wave measurement apparatus, an acoustic wave probe is used which irradiates a test object or site (hereinafter, simply referred to as an object) with an acoustic wave, receives a reflected wave (echo) therefrom, and outputs a signal. The electric signal which is converted from the reflected wave received by this acoustic wave probe is displayed as an image. As a result, an inside of the test object is visualized and observed.

As the acoustic wave, an ultrasonic wave, a photoacoustic wave, or the like having an appropriate frequency is selected according to the test object or the measurement conditions or the like.

For example, an ultrasound diagnostic apparatus transmits an ultrasonic wave to the inside of the test object, receives the ultrasonic wave reflected by tissues inside the test object, and displays the received ultrasonic wave as an image. A photoacoustic wave measurement apparatus receives an acoustic wave radiated from the inside of the test object due to a photoacoustic effect, and displays the received acoustic wave as an image. The photoacoustic effect is a phenomenon in which an acoustic wave (typically, an ultrasonic wave) is generated through thermal expansion after a test object absorbs an electromagnetic wave to generate heat in a case where the test object is irradiated with an electromagnetic wave pulse of visible light, near infrared light, microwave, or the like.

Since the acoustic wave measurement apparatus transmits and receives an acoustic wave to and from a living body which is the test object, for example, the acoustic wave measurement apparatus is required to have matching of acoustic impedance with a living body (typically a human body), and is required to suppress the amount of acoustic wave attenuation.

For example, an ultrasound diagnostic apparatus probe (also referred to as an ultrasound probe) which is one type of an acoustic wave probe includes a piezoelectric element which transmits and receives ultrasound waves, and an acoustic lens which is a portion in contact with a living body. An ultrasonic wave oscillated from the piezoelectric element is incident on the living body after being transmitted through the acoustic lens. In a case where a difference between an acoustic impedance (density $\times$ acoustic velocity) of the acoustic lens and an acoustic impedance of the living body is large, the ultrasound wave is reflected on a surface of the living body so that the ultrasound wave is not efficiently incident into the living body. As a result, it is difficult to obtain a favorable resolution. In addition, in order to transmit and receive the ultrasound waves with high sensitivity, it is desired to reduce the amount of ultrasound wave attenuation of the acoustic lens.

Therefore, as one of materials of the acoustic lens, a silicone resin having an acoustic impedance close to the acoustic impedance of the living body (in a case of the human body, 1.4 to 1.7 $\times$ $10^6$ $kg/m^2/sec$) and a small amount of ultrasound wave attenuation is used. In addition, in order to reduce a difference in acoustic impedance between the piezoelectric element and the acoustic lens and to efficiently transmit and receive the ultrasonic wave, an acoustic matching layer is usually provided between the piezoelectric element and the acoustic lens.

In addition, in the use of the acoustic wave measurement apparatus, the acoustic wave probe is rubbed against the living body to transmit and receive the acoustic waves. Therefore, the acoustic lens is required to have high mechanical strength as a portion which is rubbed against the living body and sometimes pressed against the living body. Since the silicone resin is generally soft and has deteriorated mechanical strength, it is required to improve the mechanical strength by mixing a mineral filler with the silicone resin or introducing a crosslinking structure.

For example, WO2017/130890A discloses a composition for an acoustic wave probe, the composition containing a polysiloxane mixture which contains a polysiloxane having a vinyl group, a polysiloxane having two or more Si-H groups in a molecular chain, and surface-treated silica particles having an average primary particle diameter of more than 16 nm and less than 100 nm.

In addition, JP2005-103294A discloses an ultrasound probe of an elevation angle focusing type, including an array of Micromachined Ultrasound Transducer (MUT) cells, in which a fixing material layer containing a fixing agent consisting of a silicate, and an organometallic compound or a reactive organosilane is provided between the array and a lens made of room temperature-curable silicone rubber.

## SUMMARY OF THE INVENTION

An acoustic wave probe used for examining or treating a human body is usually used in direct contact with the human body. For example, a body cavity probe used for an intracavitary treatment such as a transvaginal, transrectal, and transesophageal treatment is highly likely to come into contact with a mucous membrane, and is likely to come into contact with a sterile tissue or blood. **In** addition, a body surface probe used for scanning the abdomen, the pelvis, or the like is likely to come into contact with a sterile tissue, a skin with a wound, a mucous membrane, or a skin without a wound, depending on the processing.

Therefore, the acoustic wave probe is required to have a high level of cleanliness exceeding disinfection, in order to prevent bacterial infection. As such a sterilization treatment, a sterilization treatment with ethylene oxide gas (EOG) capable of performing a low temperature treatment has been widely performed, and the application of a hydrogen peroxide plasma treatment has also been desired.

As a result of the studies conducted by the present inventor, it has been found that, in the acoustic wave probe in which the acoustic lens is directly in contact with the acoustic matching layer, as disclosed in WO2017/130890A, adhesiveness between the acoustic matching layer and the acoustic lens is low; and even in the ultrasound probe in which the fixing material layer containing the fixing agent consisting of a silicate, and an organometallic compound or a reactive organosilane is provided between the acoustic matching layer and the acoustic lens, as disclosed in JP2005-103294A, the adhesiveness between the acoustic matching layer and the acoustic lens is reduced in a case where the ultrasound probe is repeatedly subjected to the hydrogen peroxide plasma sterilization treatment. The decrease in adhesiveness causes a decrease in performance of the acoustic wave probe. Therefore, the acoustic wave probe is required to have high sterilization durability.

An object of the present invention is to provide an acoustic wave probe having excellent adhesiveness and excellent sterilization durability, and an acoustic wave measurement apparatus or an ultrasound diagnostic apparatus including the acoustic wave probe.

As a result of intensive studies in consideration of the above-described objects, the present inventor has found that the above-described objects can be achieved by providing an interlayer between a substrate such as an acoustic matching layer, and an acoustic lens layer, forming the acoustic matching layer as a layer obtained by curing room temperature-curable silicone including a constitutional unit having a phenyl group, and forming the interlayer as a layer containing a siloxane compound, in which the siloxane compound has a configuration including a constitutional component described from a hydrolyzed silicate compound. The present invention has been completed through further studies based on these findings.

The above-described objects have been achieved by the following methods.

<1> An acoustic wave probe comprising:

a substrate;
an interlayer on the substrate; and
an acoustic lens layer on the interlayer,
in which the interlayer is a layer containing a siloxane compound,
the siloxane compound has a constitutional component derived from a hydrolyzed silicate compound, and
the acoustic lens layer is a layer obtained by curing room temperature-curable silicone, in which the room temperature-curable silicone includes a constitutional unit having a phenyl group.

<2> The acoustic wave probe according to <1>,
in which the substrate contains at least one of iron, a nonferrous metal, an inorganic material other than metal, or an organic material.
<3> The acoustic wave probe according to <2>,
in which the nonferrous metal includes at least one of aluminum, titanium, magnesium, nickel, copper, lead, zinc, tin, chromium, tungsten, cobalt, or an alloy of at least two of these metals.
<4> The acoustic wave probe according to <2> or <3>,
in which the inorganic material other than metal includes at least one of glass, ceramics, or graphite.
<5> The acoustic wave probe according to any one of <2> to <4>,
in which the organic material includes at least one of a thermoplastic resin or a thermosetting resin.
<6> The acoustic wave probe according to <5>,
in which the thermoplastic resin includes at least one of an acrylic resin, a polycarbonate resin, a styrene-based resin, or a polyphenylene sulfide resin.
<7> The acoustic wave probe according to <5> or <6>,
in which the thermoplastic resin is a crosslinked thermoplastic resin.

<8> The acoustic wave probe according to any one of <1> to <7>,
in which the substrate is a substrate in which a surface on a side of the interlayer has been subjected to a hydrophilization treatment.
<9> The acoustic wave probe according to <8>,
in which the substrate is a substrate in which the surface on the side of the interlayer has been subjected to at least one treatment of an ultraviolet treatment, an ozone treatment, a plasma treatment, or a corona treatment.
<10> The acoustic wave probe according to any one of <1> to <9>,
in which the interlayer includes a constitutional component derived from at least one of a silane coupling agent, a titanium alkoxide compound, an aluminum alkoxide compound, or a zirconium alkoxide compound.
<11> The acoustic wave probe according to any one of <1> to <10>,
in which the room temperature-curable silicone is a silicone composition containing vinyl silicone and hydrosilicone.
<12> The acoustic wave probe according to <11>,
in which the silicone composition contains surface-treated oxide particles.
<13> The acoustic wave probe according to <12>,
in which the silicone composition contains surface-treated spherical silica particles.
<14> The acoustic wave probe according to any one of <1> to <13>,
in which the acoustic wave probe is an ultrasound probe.
<15> An acoustic wave measurement apparatus comprising:
the acoustic wave probe according to any one of <1> to <13>.
<16> An ultrasound diagnostic apparatus comprising:
the acoustic wave probe according to <14>.

In the present invention, "metal alkoxide compound (for example, an aluminum alkoxide compound, a zirconium alkoxide compound, or a titanium alkoxide compound described later)" means a compound having a structure in which at least one alkoxy group is bonded to a metal atom. The alkoxy group may have a substituent. The substituent may be monovalent or divalent (for example, an alkylidene group). In addition, two alkoxy groups bonded to one metal atom may be bonded to each other to form a ring.

In the present invention, in a case of a plurality of substituents, linking groups, and the like (hereinafter, referred to as a substituent and the like) represented by a specific reference numeral, or in a case of simultaneously or alternatively defining a plurality of the substituent and the like, it means that each of the substituent and the like may be the same or different from each other. In addition, unless specified otherwise, in a case where a plurality of substituents or the like are adjacent to each other, the substituents may be linked or fused to each other to form a ring.

In the present invention, with regard to a substituent (the same applies to a linking group) in which whether it is substituted or unsubstituted is not specified, within a range not impairing the desired effect, it means that the group may have an optional substituent. The same is applied to a compound which is not specified regarding whether to be substituted or unsubstituted.

In the present invention, in a case of defining the number of carbon atoms in a group, the number of carbon atoms means the number of carbon atoms in the entire group. That is, in a case of a form in which the group has a substituent, it means the total number of carbon atoms including the substituent.

In the present invention, "Si-H group" and "Si-OH group" mean a group having three bonding sites, which are not described, on a silicon atom. In addition, [-Si-O] means a group having two bonding sites, which are not described, on a silicon atom.

In the present invention, "reactive functional group" is used in a broader sense than usual. That is, the term "reactive functional group" means a group which forms a covalent bond or the like by a reaction with another group, and a group which causes an interaction (ionic interaction, hydrogen bond, or the like) with another group. In the present invention, an unsubstituted alkoxy group is not a structure having the reactive functional group.

In addition, in the present invention, a numerical range represented by using "to" means a range including numerical values described before and after "to" as a lower limit value and an upper limit value.

The acoustic wave probe according to the aspect of the present invention has excellent initial adhesiveness and excellent sterilization durability. In addition, the acoustic wave measurement apparatus and the ultrasound diagnostic apparatus according to the aspects of the present invention include the acoustic wave probe having excellent performance described above.

BRIEF DESCRIPTION OF THE DRAWINGS

Fig. 1 is a perspective view of an example of a convex type ultrasound probe which is an aspect of an acoustic wave probe.

## DESCRIPTION OF THE PREFERRED EMBODIMENTS

<<Acoustic wave probe>>

The acoustic wave probe according to the embodiment of the present invention includes a substrate, an interlayer on the substrate, and an acoustic lens layer on the interlayer, in which the interlayer is a layer containing a siloxane compound, the siloxane compound has a constitutional component derived from a hydrolyzed silicate compound, and the acoustic lens layer is a layer obtained by curing room temperature-curable silicone, in which the room temperature-curable silicone includes a constitutional unit having a phenyl group.

The acoustic wave probe according to the embodiment of the present invention has excellent initial adhesiveness and excellent sterilization durability. The reason for this is not clear, but since the siloxane compound contained in the interlayer has a constitutional component derived from a hydrolyzed silicate compound, and the acoustic lens layer is a layer obtained by curing the room temperature curable silicone, it is considered that a chemical bond is formed at an interface between the interlayer and the acoustic lens layer due to a reaction between a hydrophilic group (silanol group [Si-OH]) included in the hydrolyzed silicate compound and a hydrosilyl group [Si-H] in the room temperature-curable silicone. Furthermore, since the acoustic lens layer in the present invention is a layer formed by curing the room temperature-curable silicone including a constitutional unit having a phenyl group, it is considered that the acoustic lens layer can suppress transmission of hydrogen peroxide gas, an activator, and the like (hereinafter, abbreviated as "hydrogen peroxide gas and the like") generated by the hydrogen peroxide gas and plasma irradiation, even in a case where the acoustic lens layer is subjected to a hydrogen peroxide plasma sterilization treatment having a strong chemical force, due to the phenyl group having a large molecular size present in the acoustic lens layer, and can also reduce the amount of the hydrogen peroxide gas and the like reaching the interface between the acoustic lens layer and the interlayer, and an interface between the interlayer and the substrate such as an acoustic matching layer. Since the hydrogen peroxide gas and the like may cleave a chemical bond which contributes to adhesion of an organic component, particularly a material in the vicinity of the interface, the configuration of the present invention can exhibit excellent hydrogen peroxide plasma sterilization resistance.

<Substrate>

The substrate in the acoustic wave probe according to the embodiment of the present invention is not particularly limited, and a substrate used for a constituent member around the acoustic lens layer, such as an acoustic matching layer in the acoustic wave probe, can be widely adopted.

That is, the acoustic wave probe according to the embodiment of the present invention has a configuration in which a constituent member positioned around the acoustic lens layer in a general acoustic wave probe is adhered to the above-described acoustic lens layer through the above-described interlayer. Among the above-described constituent members around the acoustic lens layer, a constituent member adhered to the above-described acoustic lens layer by the above-described interlayer is the substrate in the acoustic wave probe according to the embodiment of the present invention.

The acoustic matching layer is suitable as the above-described substrate, and it is preferable that the acoustic matching layer is adhered to the above-described acoustic lens layer through the above-described interlayer.

It is preferable that the above-described substrate contains at least one of iron, a nonferrous metal, an inorganic material other than metal, or an organic material.

In the present invention, among metal materials, a material containing an iron element is classified as the iron, and a material not containing an iron element is classified as the nonferrous metal.

That is, the above-described iron includes not only pure iron consisting of an iron element, but also an alloy of an iron element and a nonferrous metal and/or a non-metal element. Examples of such an alloy containing an iron element include stainless steel.

Examples of the above-described nonferrous metal include aluminum, titanium, magnesium, nickel, copper, lead, zinc, tin, chromium, tungsten, cobalt, vanadium, gold, or an alloy of at least two of these metals.

From the viewpoint of more excellent hydrogen peroxide plasma sterilization resistance, the above-described metal material is preferably the nonferrous metal.

From the viewpoint of low catalytic action on hydrogen peroxide, the above-described nonferrous metal preferably includes at least one of aluminum, titanium, magnesium, nickel, copper, lead, zinc, tin, chromium, tungsten, cobalt, or an alloy of at least two of these metals; more preferably includes at least one of aluminum, titanium, nickel, copper, chromium, tungsten, cobalt, or an alloy of at least two of these metals; still more preferably includes at least one of copper, aluminum, tungsten, or an alloy of at least two of these metals; and particularly preferably includes at least one of copper, aluminum, or an alloy of these metals.

Examples of the above-described inorganic material other than metal include glass, ceramics, and graphite, and

it is preferable that the inorganic material includes at least one of glass, ceramics, or graphite.

Examples of the above-described glass include sodium soda glass, Pyrex (registered trademark) glass, quartz glass, and non-alkali glass.

The above-described ceramics means an inorganic compound such as an oxide, a carbide, a nitride, or a boride of at least one element of the metal element or the non-metal element; and examples thereof include alumina, zirconia, silicon carbide, and silicon nitride.

Examples of the above-described graphite include natural graphite and artificial graphite.

The above-described organic material is usually a solid material, and preferably includes at least one of a thermoplastic resin or a thermosetting resin.

Examples of the above-described thermoplastic resin include polyolefin resins such as a polyethylene resin, a polypropylene resin, and a polymethylpentene resin; styrene-based resins such as a polystyrene resin, an acrylonitrile-butadiene-styrene copolymer resin (ABS resin), and an acrylonitrile-styrene copolymer resin; an acrylic resin; a thermoplastic polyimide resin; a thermoplastic polyamide resin; polyester resins such as a polyetherimide resin, a polyphenylene ether resin, a polycarbonate resin, a polyethylene terephthalate resin, a polybutylene terephthalate resin, and a polyethylene naphthalate resin; a polyphenylene sulfide resin; a polyether ether ketone resin; a polyether sulfone resin; and thermoplastic polycycloolefin resins such as a thermoplastic polynorbornene resin.

From the viewpoint of excellent secondary workability such as cutting and machining for forming an ultrasound probe, the above-described thermoplastic resin preferably includes at least one of an acrylic resin, a polycarbonate resin, a styrene-based resin, or a polyphenylene sulfide resin. Among these, from the viewpoint of further improving the hydrogen peroxide plasma sterilization resistance and heat cycle resistance, the above-described thermoplastic resin more preferably includes a crosslinked thermoplastic resin, and still more preferably includes a crosslinked polystyrene-based resin.

The crosslinked thermoplastic resin may be a resin in which a crosslinking structure is introduced into the thermoplastic resin, and the crosslinking structure can be introduced by a conventional method without particular limitation.

Examples of the above-described thermosetting resin include thermosetting polycycloolefin resins such as an epoxy resin, a phenol resin, a melamine resin, a urethane resin, a thermosetting polyimide resin, a thermosetting polyamide resin, a polyamideimide resin, an unsaturated polyester resin, and a thermosetting polynorbornene resin.

The above-described unsaturated polyester resin refers to a resin obtained by dissolving an unsaturated polyester resin which is generated by polycondensation of an unsaturated dicarboxylic acid such as fumaric acid and maleic acid anhydride and a dihydric alcohol such as ethylene glycol, in a polymerizable monomer such as styrene and methyl methacrylate.

From the viewpoint of further enhancing the hydrogen peroxide plasma sterilization resistance, the thermosetting resin preferably includes at least one of a phenol resin, an unsaturated polyester resin, a thermosetting polyimide resin, a thermosetting polyamide resin, a polyamideimide resin, or a thermosetting polycycloolefin resin; and more preferably includes at least one of an unsaturated polyester resin, a thermosetting polyimide resin, or a thermosetting polyamideimide resin.

From the viewpoint of further enhancing the hydrogen peroxide plasma sterilization resistance and heat cycle resistance, the above-described organic material preferably includes at least one of a thermosetting resin or a crosslinked thermoplastic resin; and more preferably includes at least one of a thermosetting polyimide resin, an unsaturated polyester resin, or a crosslinked polystyrene resin.

From the viewpoint of further enhancing the hydrogen peroxide plasma sterilization resistance, the above-described substrate preferably contains at least one of the nonferrous metal, the inorganic material other than metal, or the organic material; from the viewpoint of further enhancing the heat cycle resistance in addition to the hydrogen peroxide plasma sterilization resistance, the above-described substrate more preferably contains at least one of the inorganic material other than metal or the organic material; and from the viewpoint of further enhancing the hydrogen peroxide plasma sterilization resistance and the heat cycle resistance, the above-described substrate still more preferably contains at least one of the thermosetting resin or the crosslinked thermoplastic resin.

The total content of at least one of the iron, the nonferrous metal, the inorganic material other than metal, or the organic material contained in the above-described substrate is not particularly limited, and can be, for example, 80% by mass or more, preferably 90% by mass or more, and may be 100% by mass.

In a case where the iron, the nonferrous metal, the inorganic material other than metal, and the organic material contained in the above-described substrate are limited to specific materials, the total content of the limited materials can be, for example, 80% by mass or more, preferably 90% by mass or more, and may be 100% by mass. Therefore, in a case where the above-described substrate contains the iron and the organic material, does not contain the nonferrous metal, and does not contain the inorganic material other than metal, the total content of the iron and the organic material in the substrate can be set to the above-described preferred content.

From the viewpoint of improving the adhesiveness between the substrate and the interlayer, it is preferable that

the above-described substrate is a substrate in which a surface on a side of the interlayer has been subjected to a hydrophilization treatment. Since the surface of the substrate, subjected to the hydrophilization treatment, has many reaction points (hydrophilic groups) with the siloxane compound having a constitutional component derived from a hydrolyzed silicate compound, contained in the interlayer, a peel strength can be further improved, and the peel strength can be maintained at an excellent level even after heat cycle and hydrogen peroxide plasma sterilization.

The above-described hydrophilization treatment is not particularly limited as long as the reaction point with the siloxane compound having a constitutional component derived from a hydrolyzed silicate compound, contained in the interlayer, can be imparted (introduced) to the surface of the substrate; and examples thereof include at least one treatment of an ultraviolet (UV) treatment, an ozone treatment, a plasma treatment, or a corona treatment. In a case where two or more of these treatments are performed, the two or more treatments may be performed at the same time or separately.

The UV treatment, the ozone treatment, the plasma treatment, and the corona treatment can be performed by a conventional method as long as the effects of the present invention are not impaired.

From the viewpoint of further enhancing the heat cycle resistance and the hydrogen peroxide plasma sterilization resistance, the hydrophilization treatment is preferably at least one treatment of a UV treatment, a plasma treatment, or a corona treatment; and more preferably at least one treatment of a UV treatment or a plasma treatment.

<Interlayer>

The interlayer in the acoustic wave probe according to the embodiment of the present invention is a layer containing a siloxane compound, and the siloxane compound has a constitutional component derived from a hydrolyzed silicate compound.

The siloxane compound is a compound having a repeating structure of a siloxane bond ([-Si-O]), and is an oligomer or a polymer obtained by polycondensation between silanol groups (Si-OH groups) contained in the hydrolyzed silicate compound.

The above-described hydrolyzed silicate compound is a compound obtained by hydrolyzing a hydrolyzable group in a silicate compound having a hydrolyzable group to convert the hydrolyzable group into a silanol group.

The silicate compound means a compound having a structure in which four O atoms are coordinated around one Si atom, and a plurality of Si atoms each coordinated with four O atoms may be present in the compound. A compound in which the number of Si atoms coordinated with four O atoms is 1 is referred to as a silicate monomer (also referred to as a silane compound), and in which the number of Si atoms coordinated with four O atoms is 2 or more is referred to as a silicate oligomer.

In the present invention, the hydrolyzed silicate compound is preferably a hydrolyzed silicate oligomer.

A hydrolysis rate of the hydrolyzed silicate compound is not particularly limited as long as the effects of the present invention can be obtained, and it may be, for example, 50% or more, preferably 60% or more, more preferably 70% or more, and still more preferably 80% or more. In a case where the silicate compound having a hydrolyzable group is hydrolyzed in the presence of an acid or a base as described later, a hydrolyzed silicate compound having a hydrolysis rate corresponding to the amount of water to be used can be usually obtained. A hydrolysis rate of a commercially available product or a hydrolyzed silicate compound obtained by hydrolysis, which is used in Examples described later, is 80% or more.

The hydrolysis rate of the hydrolyzed silicate compound is a value calculated from the following calculation expression using the number $X_H$ of hydrolyzable groups included in the hydrolyzed silicate compound and the number $X_{OH}$ of hydroxy groups in the silanol group. The number $X_{OH}$ of hydroxy groups in $-Si(OH)_3$ is calculated as 3.

$$\text{Hydrolysis rate of hydrolyzed silicate compound} = 100\% \times X_{OH}/(X_H + X_{OH})$$

As the hydrolyzed silicate compound, a commercially available product may be purchased and used, and for example, Colcoat PX, Colcoat N-103X, HAS-1, HAS-6, and HAS-10 (all of which are trade names) which are hydrolyzed silicate solutions manufactured by Colcoat Co,.Ltd. can be used.

In addition, a compound obtained by hydrolyzing a silicate compound having a hydrolyzable group can also be used as the hydrolyzed silicate compound.

The hydrolysis reaction can be promoted by reacting the silicate compound having a hydrolyzable group in the presence of an acid or a base as a catalyst. On the other hand, under neutral conditions in which neither an acid nor a base is present, the hydrolysis reaction hardly proceeds.

As the acid or base used as the catalyst, an acid or a base, which is usually used in a hydrolysis reaction of a silicate compound, can be used without particular limitation.

Among these, from the viewpoint that gelation is likely to proceed in a case where the base is used as the catalyst,

an acid catalyst is preferably used, and hydrochloric acid is more preferably used.

The amount of water used for the hydrolysis reaction can be appropriately adjusted according to the desired hydrolysis rate.

As a solvent used in the hydrolysis reaction, a solvent in which the silicate compound having a hydrolyzable group and water are soluble can be used, and it is preferable to use one kind or two or more kinds of alcohol solvents such as methanol, ethanol, isopropanol, and ethyl cellosolve.

A reaction time of the hydrolysis reaction can be appropriately adjusted, and for example, it can be set to 1 to 24 hours.

Examples of the hydrolyzable group include an alkoxy group (alkyloxy group), an alkenyloxy group, an acyloxy group, an aminooxy group, an oxime group, and an amide group; and an alkoxy group is preferable.

An alkyl group in the alkoxy group may be linear, branched, or cyclic. The number of carbon atoms in the alkyl group is preferably 1 to 30, more preferably 1 to 20, still more preferably 1 to 10, and particularly preferably 1 or 2. Specific examples of the alkyl group include methyl, ethyl, isopropyl, butyl, and cyclopentyl.

**In** a case where the acyloxy group, the aminooxy group, the oxime group, or the amide group is substituted with an alkyl group, the description of the alkyl group in the alkoxy group described above can be applied to the alkyl group in the acyloxy group, the aminooxy group, the oxime group, or the amide group.

An alkenyl group in the alkenyloxy group may be linear, branched, or cyclic. The number of carbon atoms in the alkenyl group is preferably 2 to 30, more preferably 2 to 20, and still more preferably 2 to 10.

Examples of the silicate monomer in the silicate compound having a hydrolyzable group include a tetraalkoxysilane compound.

The above-described tetraalkoxysilane compound is not particularly limited, and examples thereof include tetramethoxysilane, tetraethoxysilane, tetrapropoxysilane, tetraisopropoxysilane, and tetrabutoxysilane.

In addition, the hydrolyzed silicate compound can also be obtained by hydrolyzing a mixture of the above-described tetraalkoxysilane compound and a trialkoxysilane compound and/or a dialkoxysilane compound. The above-described trialkoxysilane compound and the above-described dialkoxysilane compound are compounds having a structure in which three or two O atoms are coordinated around one Si atom, and are not classified as the silicate compound.

As conditions for the hydrolysis reaction of the mixture, the conditions for the hydrolysis reaction of the silicate compound having a hydrolyzable group described above can be applied.

The above-described trialkoxysilane compound is not particularly limited, and examples thereof include methyltrimethoxysilane, methyltriethoxysilane, phenyltrimethoxysilane, and phenyltriethoxysilane.

The above-described dialkoxysilane compound is not particularly limited, and examples thereof include dimethyldimethoxysilane and dimethyldiethoxysilane.

Among the silicate compounds having a hydrolyzable group, the silicate oligomer can be obtained by a hydrolysis condensation reaction of the silicate monomer. Examples of the silicate oligomer in the silicate compound having a hydrolyzable group include a hydrolysis condensate of the above-described tetraalkoxysilane compound and a hydrolysis condensate of a mixture of the above-described tetraalkoxysilane compound and a trialkoxysilane compound and/or a dialkoxysilane compound.

The number of Si atoms coordinated with four O atoms in the silicate oligomer is preferably 2 to 200 and more preferably 2 to 120.

Among these, a hydrolysis condensate of tetraalkoxysilane is preferable, a hydrolysis condensate of tetramethoxysilane and/or tetraethoxysilane is more preferable, and a hydrolysis condensate of tetramethoxysilane or tetraethoxysilane is still more preferable.

As the silicate compound having a hydrolyzable group, a commercially available product may be purchased and used; and examples of the silicate monomer include Ethyl Silicate 28, N-propyl Silicate, N-butyl Silicate, and the like (all of which are trade names) manufactured by Colcoat Co,.Ltd., and examples of the silicate oligomer include Ethyl Silicate 40, Ethyl Silicate 48, Methyl Silicate 51, Methyl Silicate 53A, and EMS-485 (all of which are trade names) manufactured by Colcoat Co,.Ltd., and MKC SILICATE MS51, MS56, MS57, and MS56S (all of which are trade names) manufactured by MITSUBISHI CHEMICAL CORPORATION.

It is preferable that the hydrolyzed silicate compound does not have an organic group other than methyl and ethyl.

A weight-average molecular weight of the hydrolyzed silicate compound is not particularly limited, and is, for example, preferably 100 to 10,000, more preferably 150 to 4,000, and particularly preferably 200 to 2,500.

A weight-average molecular weight or a number-average molecular weight of a compound, described in the specification of the present application, is determined as follows.

The weight-average molecular weight or the number-average molecular weight can be measured as a polystyrene-equivalent molecular weight by gel permeation chromatography (GPC).

Specifically, a GPC device HLC-8220 (trade name, manufactured by Tosoh Corporation) is used, tetrahydrofuran is used as an eluent, G3000HXL + G2000HXL (both are trade names, manufactured by Tosoh Corporation) are used as

columns, and detection can be performed by a differential refractive index (RI) at 23°C and a flow rate of 1 mL/min.

The acoustic wave probe according to the embodiment of the present invention includes a form in which the layer containing the siloxane compound as the interlayer reacts with at least one of the substrate or the acoustic lens layer. For example, the interlayer can be present in a case where a hydroxy group included in the siloxane compound reacts with a group on the surface of the acoustic lens layer or the substrate.

A content of the siloxane compound in the interlayer is preferably 50% by mass or more, more preferably 70% by mass or more, still more preferably 80% by mass or more, and particularly preferably 90% by mass or more. The upper limit value thereof is not particularly limited, and is, for example, 100% by mass or less, preferably 99% by mass or less, and more preferably 98% by mass or less. That is, the above-described content of the siloxane compound in the interlayer is preferably 50% to 100% by mass, more preferably 70% to 99% by mass, and still more preferably 80% to 98% by mass.

A content of the hydrolyzed silicate compound in the liquid (which may contain a solvent) used for forming the interlayer is, for example, preferably 1% by mass or more, more preferably 2% by mass or more, still more preferably 3% by mass or more, and particularly preferably 4% by mass or more. The upper limit value thereof is not particularly limited, and is, for example, 100% by mass or less, preferably 70% by mass or less, more preferably 50% by mass or less, and still more preferably 25% by mass or less. That is, the above-described content of the hydrolyzed silicate compound in the liquid (containing a solvent) used for forming the interlayer is preferably 1% to 100% by mass, more preferably 2% to 70% by mass, still more preferably 3% to 50% by mass, and particularly preferably 4% to 25% by mass.

In the present invention, from the viewpoint of obtaining more excellent peeling strength, the interlayer preferably further includes a constitutional component derived from at least one of a silane coupling agent, a titanium alkoxide compound, an aluminum alkoxide compound, or a zirconium alkoxide compound; and from the viewpoint of more excellent heat cycle resistance and hydrogen peroxide plasma sterilization resistance, the interlayer more preferably further includes a constitutional component derived from an aluminum alkoxide compound.

(Silane coupling agent)

As the silane coupling agent used in the present invention, a general silane coupling agent which can be applied as a primer layer of a constituent member of the acoustic wave probe can be widely adopted.

It is preferable that the silane coupling agent does not have a siloxane bond, and it is preferable that the silane coupling agent has a group other than methyl, ethyl, methoxy, and ethoxy (for example, an amino group, a vinyl group, a propyl group, an acid anhydride group, an epoxy group, and a mercapto group).

(Titanium alkoxide compound)

As the titanium alkoxide compound (preferably, a titanium coupling agent) used in the present invention, a general titanium alkoxide compound which can be applied as a primer layer of a constituent member of the acoustic wave probe can be widely adopted.

The titanium alkoxide compound preferably includes at least one compound represented by General Formula (a) or (b), and more preferably includes at least one compound represented by General Formula (a). A proportion of the total content of the compound represented by General Formula (a) or (b) in the above-described titanium alkoxide compound is not particularly limited, and for example, it can be set to 60% by mass or more, preferably 80% by mass or more, more preferably 90% by mass or more, still more preferably 95% by mass or more, and may be 100% by mass.

General Formula (a): $R^{1a}{}_{m1}\text{-Ti-}(OR^{2a})_{4-m1}$

General Formula (b): $O\text{-}[Ti\text{-}(OR^{2a})_3]_2$

$R^{1a}$ represents a hydrogen atom, an alkyl group, a cycloalkyl group, an acyl group, an aryl group, or an unsaturated aliphatic group.

As the alkyl group which can be adopted as $R^{1a}$, the cycloalkyl group, the acyl group, the aryl group, and the unsaturated aliphatic group, for example, an alkyl group, a cycloalkyl group, an acyl group, an aryl group, and an unsaturated aliphatic group which can be adopted as $R^{1b}$ of General Formula (c) described later can be adopted.

$R^{2a}$ represents a hydrogen atom, an alkyl group, a cycloalkyl group, an acyl group, an alkenyl group, an aryl group, a phosphonate group, or $-SO_2R^{S1}$. $R^{S1}$ represents a substituent.

As the alkyl group, the cycloalkyl group, the acyl group, the alkenyl group, the aryl group, and the phosphonate group, which can be adopted as $R^{2a}$, for example, the alkyl group, the cycloalkyl group, the acyl group, the alkenyl group, the aryl group, and the phosphonate group, which can be adopted as $R^{2b}$ in General Formula (c) described later, can be applied. In addition, as the substituent which can be adopted as $R^{S1}$, for example, a substituent which can be adopted as $R^{S2}$ of General Formula (c) described later can be applied.

m1 is an integer of 0 to 3.

The compound represented by General Formula (a) or (b) preferably contains at least one atom of N, P, or S.

In a case where the compound represented by General Formula (a) or (b) has N, it is preferable to have N as an amino group.

In a case where the compound represented by General Formula (a) or (b) has P, it is preferable to have P as a phosphate group (phosphoric acid group) or a phosphonate group (phosphonic acid group).

In a case where the compound represented by General Formula (a) or (b) has S, it is preferable to have S as a sulfonyl group ($-SO_2-$).

In addition, it is also preferable that the compound represented by General Formula (a) or (b) has an acyl group as $R^{2a}$, that is, has an acetato structure described later as $OR^{2a}$.

Hereinafter, specific examples of the titanium alkoxide compound used in the present invention will be described, but the present invention is not limited thereto.

Isopropyltriisostearoyl titanate
Isopropyltridodecylbenzenesulfonyl titanate
Isopropyltrioctanoyl titanate
Isopropyltri(dioctylphosphite)titanate
Isopropyltris(dioctylpyrophosphate)titanate
Isopropyltri(dioctylsulfate)titanate
Isopropyltricumylphenyl titanate
Isopropyltri(N-aminoethyl-aminoethyl)titanate
Isopropyldimethacryl isostearoyl titanate
Isopropylisostearoyl diacryl titanate
Isobutyltrimethyl titanate
Diisostearoylethylene titanate
Diisopropyl bis(dioctylpyrophosphate)titanate
Dioctyl bis(ditridecylphosphate)titanate
Dicumyl phenyl oxyacetate titanate
Bis(dioctylpyrophosphate)oxyacetate titanate
Bis(dioctylpyrophosphate)ethylene titanate
Tetraisopropyl titanate
Tetrabutyl titanate
Tetraoctyl titanate
Tetrastearyl titanate
Tetraisopropyl bis(dioctylphosphite)titanate
Tetraoctyl bis(di-tridecylphosphite)titanate
Tetra(2,2-diallyloxymethyl-1-butyl)bis(di-tridecyl)phosphite titanate
Butyl titanate dimer
Titanium tetraacetylacetonate
Titanium ethyl acetoacetate
Titanium octylene glycolate
Titanium di-2-ethylhexoxybis(2-ethyl-3-hydroxyhexoxide)

(Aluminum alkoxide compound)

As the aluminum alkoxide compound (preferably, an aluminum coupling agent) used in the present invention, a general aluminum alkoxide compound which can be applied as a primer layer of a constituent member of the acoustic wave probe can be widely employed.

The aluminum alkoxide compound preferably includes at least one compound represented by General Formula (c) or (d), and more preferably includes at least one compound represented by General Formula (c). A proportion of the total content of the compound represented by General Formula (c) or (d) in the above-described aluminum alkoxide compound is not particularly limited, and for example, it can be set to 60% by mass or more, preferably 80% by mass or more, more preferably 90% by mass or more, still more preferably 95% by mass or more, and may be 100% by mass.

General Formula (c): $R^{1b}_{m2}\text{-Al-}(OR^{2b})_{3-m2}$

General Formula (d): $O\text{-}[Al\text{-}(OR^{2b})_2]_2$

$R^{1b}$ represents a hydrogen atom, an alkyl group, a cycloalkyl group, an acyl group, an aryl group, or an unsaturated aliphatic group.

The alkyl group which can be adopted as $R^{1a}$ includes a linear alkyl group, a branched alkyl group, and an aralkyl group. The number of carbon atoms in the alkyl group is preferably 1 to 20, more preferably 1 to 15, still more preferably 1 to 10, and particularly preferably 1 to 8, and in a case of an aralkyl group, the number of carbon atoms in the alkyl group is preferably 7 to 30. Preferred specific examples of the alkyl group include methyl, ethyl, propyl, isopropyl, butyl, sec-butyl, isobutyl, tert-butyl, pentyl, hexyl, heptyl, octyl, decyl, tridecyl, octadecyl, benzyl, and phenethyl.

It is also preferable that the alkyl group which can be adopted as $R^{1b}$ has an oxirane ring, and examples of $R^{1b}$ include an epoxycycloalkyl group. The number of ring members in the cycloalkyl group (cycloalkyl group in which oxacyclopropane as an oxirane ring is condensed) in the epoxycycloalkyl group which can be adopted as $R^{1b}$ is preferably 4 to 8, more preferably 5 or 6, and still more preferably 6 (that is, the epoxycycloalkyl group is an epoxycyclohexyl group).

In addition, the alkyl group which can be adopted as $R^{1a}$ preferably has a group selected from an amino group, an isocyanato group, a mercapto group, an ethylenic unsaturated group, and an acid anhydride group.

The number of carbon atoms in the cycloalkyl group which can be adopted as $R^{1b}$ is preferably 3 to 20, more preferably 3 to 15, still more preferably 3 to 10, and particularly preferably 3 to 8. Preferred specific examples of the cycloalkyl group include cyclopropyl, cyclopentyl, and cyclohexyl.

The number of carbon atoms in the acyl group which can be adopted as $R^{1b}$ is preferably 2 to 40, more preferably 2 to 30, still more preferably 2 to 20, and particularly preferably 2 to 18.

The number of carbon atoms in the aryl group which can be adopted as $R^{1b}$ is preferably 6 to 20, more preferably 6 to 15, still more preferably 6 to 12, and particularly preferably 6 to 10. Preferred specific examples of the aryl group include phenyl and naphthyl, among which phenyl is even still more preferable.

The number of carbon-carbon unsaturated bonds in the unsaturated aliphatic group which can be adopted as $R^{1b}$ is preferably 1 to 5, more preferably 1 to 3, still more preferably 1 or 2, and particularly preferably 1. The unsaturated aliphatic group may contain a heteroatom, and is also preferably a hydrocarbon group. In a case where the unsaturated aliphatic group is a hydrocarbon group, the number of carbon atoms in the group is preferably 2 to 20, more preferably 2 to 15, still more preferably 2 to 10, even still more preferably 2 to 8, and is also preferably 2 to 5. The unsaturated aliphatic group is more preferably an alkenyl group or an alkynyl group.

$R^{1b}$ is preferably a hydrogen atom, an alkyl group, a cycloalkyl group, or an aryl group, and more preferably an alkyl group or a cycloalkyl group.

In a case where the compound of General Formula (c) has two or more $R^{1b}$'s, the two $R^{1b}$'s may be linked to each other to form a ring.

$R^{2b}$ represents a hydrogen atom, an alkyl group, a cycloalkyl group, an acyl group, an alkenyl group, an aryl group, a phosphonate group (phosphonic acid group), or $-SO_2R^{S2}$. $R^{S2}$ represents a substituent.

The alkyl group, cycloalkyl group, acyl group, and aryl group which can be adopted as $R^{2b}$ have the same definition as the alkyl group, cycloalkyl group, acyl group, and aryl group which can be adopted as $R^{1b}$, respectively, and a preferred form of each group is also the same as in $R^{1b}$. In addition, the alkyl group which can be adopted as $R^{2b}$ preferably has an amino group as a substituent.

The alkenyl group which can be adopted as $R^{2b}$ includes a linear alkenyl group and a branched alkenyl group. The number of carbon atoms in the alkenyl group is preferably 2 to 18, more preferably 2 to 7, and still more preferably 2 to 5. Preferred specific examples of the alkenyl group include vinyl, allyl, butenyl, pentenyl, and hexenyl. The alkenyl group is preferably a substituted alkenyl group.

The phosphonate group which can be adopted as $R^{2b}$ is a group represented by $-P(=O)(-OR^{P1})OR^{P2}$. $R^{P1}$ and $R^{P2}$ represent a hydrogen atom or a substituent, and the substituent is preferably an alkyl group or a phosphonate group. The alkyl group which can be adopted as $R^{P1}$ and $R^{P2}$ has the same definition as the alkyl group which can be adopted as $R^{1b}$ described above, and a preferred form of the alkyl group is also the same as in $R^{1b}$. The phosphonate group which can be adopted as $R^{P1}$ and $R^{P2}$ has the same definition as the phosphonate group which can be adopted as $R^{2b}$, and a preferred form thereof is also the same as in $R^{2b}$. In a case where $R^{P1}$ or $R^{P2}$ is a phosphonate group, the $R^{P1}$ and $R^{P2}$ constituting the phosphonate group are each preferably an alkyl group.

In the phosphonate group which can be adopted as $R^{2b}$, it is preferable that both $R^{P1}$ and $R^{P2}$ are alkyl groups, or $R^{P1}$ is a hydrogen atom and $R^{P2}$ is a phosphonate group.

Since the phosphonate group is tautomeric with a phosphite group (phosphorous acid group), the phosphonate group in the present invention means to include the phosphite group.

In $-SO_2R^{S2}$ which can be adopted as $R^{2b}$, the substituent $R^{S2}$ is preferably an alkyl group or an aryl group. Preferred forms of the alkyl group and aryl group which can be adopted as $R^{S2}$ include the above-described preferred forms of the alkyl group and aryl group which can be adopted as $R^{1b}$, respectively. Among these, phenyl having an alkyl group as a substituent is preferable for $R^{S2}$. A preferred form of the alkyl group is the same as the above-described preferred form of the alkyl group which can be adopted as $R^{1b}$.

In a case where the compound represented by General Formula (c) has two or more $R^{2b}$'s, the two $R^{2b}$'s may be

linked to each other to form a ring. In the compound represented by General Formula (d), two $R^{2b}$'s may be linked to each other to form a ring.

m2 is an integer of 0 to 2.

In General Formulae (c) and (d), it is preferable that at least one of $OR^{2b}$'s has an acetonato structure. The acetonato structure means a structure that one hydrogen ion is removed from acetone or a compound having a structure in which acetone has a substituent, and then the resultant is coordinated to Al. A coordinating atom coordinated to the Al is usually an oxygen atom. The acetonato structure is preferably a structure in which an acetylacetone structure ("$CH_3$-C(=O)-$CH_2$-C(=O)-$CH_3$") is taken as a basic structure, one hydrogen ion is removed from the structure, and the structure is coordinated to Al through an oxygen atom as a coordinating atom (that is, an acetylacetonato structure). The phrase "acetylacetone structure is taken as a basic structure" means to include a structure in which a hydrogen atom of the acetylacetone structure is substituted with a substituent, in addition to the above-described acetylacetone structure. Examples of the form in which $OR^{2b}$ has an acetonato structure include compounds AL-1 and AL-2 described later.

In addition, in General Formulae (c) and (d), it is preferable that at least one of $OR^{2b}$'s has an acetato structure. In the present invention, the acetato structure means a structure that one hydrogen ion is removed from acetic acid or an acetic acid ester, or a compound having a structure in which the acetic acid or acetic acid ester has a substituent (including a form in which the methyl group of acetic acid has an alkyl group as a substituent), and then the resultant is coordinated to Al. A coordinating atom coordinated to the Al is usually an oxygen atom. The acetato structure is preferably a structure in which an alkylacetoacetato structure ("$CH_3$-C(=O)-$CH_2$-C(=O)-O-$R_{alk}$" ($R_{alk}$ represents an alkyl group (preferably an alkyl group having 1 to 20 carbon atoms, may be an alkyl group having 1 to 10 carbon atoms, and more preferably an alkyl group having 1 to 4 carbon atoms))) is taken as a basic structure, one hydrogen ion is removed from the structure, and the structure is coordinated to Al through an oxygen atom as a coordinating atom (that is, an alkylacetoacetato structure). The phrase "an alkylacetoacetato structure is taken as a basic structure" means to include a structure in which a hydrogen atom of the alkylacetoacetato structure is substituted with a substituent, in addition to the above-described alkylacetoacetato structure. Examples of the form in which $OR^{2a}$ has an acetato structure include compounds AL-2, AL-3, and AL-4 described later.

The above-described group which can be adopted as $R^{1b}$ or $R^{2b}$ may have an anionic group having a counter cation (salt-type substituent) as a substituent. The anionic group means a group capable of forming an anion. Examples of the anionic group having a counter cation include a carboxylic acid ion group having an ammonium ion as a counter cation. In this case, the above-described counter cation may be present in the compound represented by General Formula (c) or (d) such that a charge of the entire compound is zero. The same applies to the compound represented by General Formula (a) or (b) described above and a compound represented by General Formula (e) or (f) described later.

Hereinafter, specific examples of the aluminum alkoxide compound used in the present invention will be given, but the present invention is not limited thereto.

Aluminum triethylate
Aluminum triisopropylate
Aluminum tri-sec-butyrate
Aluminum tris(ethylacetoacetate)
Ethyl acetoacetate aluminum diisopropylate
Aluminum monoacetylacetonate bis(ethylacetoacetate)
Aluminum tris(acetylacetonate)
Diisopropoxy aluminum-9-octadecenylacetoacetate
Aluminum diisopropoxy monoethyl acetoacetate
Mono sec-butoxyaluminum diisopropylate
Diethylacetoacetate aluminum isopropylate
Aluminum bis(ethylacetoacetate) monoacetylacetonate
Aluminum octadecylacetoacetate diisopropylate

(Zirconium alkoxide compound)

As the zirconium alkoxide compound (preferably, a zirconium coupling agent) used in the present invention, a general zirconium alkoxide compound which can be applied as a primer layer of a constituent member of the acoustic wave probe can be widely employed.

The zirconium alkoxide compound preferably includes at least one compound represented by General Formula (e) or (f), and more preferably includes at least one compound represented by General Formula (e). A proportion of the total content of the compound represented by General Formula (e) or (f) in the above-described zirconium alkoxide compound is not particularly limited, and for example, it can be set to 60% by mass or more, preferably 80% by mass or more, more preferably 90% by mass or more, still more preferably 95% by mass or more, and may be 100% by mass.

General Formula (e): $R^{1c}{}_{m3}\text{-Zr-}(OR^{2c})_{4-m3}$

General Formula (f): $O\text{-}[Zr\text{-}(OR^{2c})_3]_2$

$R^{1c}$ represents a hydrogen atom, an alkyl group, a cycloalkyl group, an acyl group, an aryl group, or an unsaturated aliphatic group.

As the alkyl group which can be adopted as $R^{1c}$, the cycloalkyl group, the acyl group, the aryl group, and the unsaturated aliphatic group, for example, an alkyl group, a cycloalkyl group, an acyl group, an aryl group, and an unsaturated aliphatic group which can be adopted as $R^{1b}$ of General Formula (c) described above can be adopted.

$R^{2c}$ represents a hydrogen atom, an alkyl group, a cycloalkyl group, an acyl group, an alkenyl group, an aryl group, a phosphonate group, or $-SO_2R^{S3}$. $R^{S3}$ represents a substituent.

As the alkyl group, the cycloalkyl group, the acyl group, the alkenyl group, the aryl group, and the phosphonate group, which can be adopted as $R^{2c}$, for example, the alkyl group, the cycloalkyl group, the acyl group, the alkenyl group, the aryl group, and the phosphonate group, which can be adopted as $R^{2b}$ in General Formula (c) described above, can be applied. In addition, as the substituent which can be adopted as $R^{S3}$, for example, a substituent which can be adopted as $R^{S2}$ of General Formula (c) described above can be applied.

m3 is an integer of 0 to 3.

In General Formulae (e) and (f), it is preferable that at least one of $OR^{2c}$'s has an acetonato structure. The acetonato structure has the same definition as the acetonato structure described by General Formula (c). Examples of the form in which $OR^{2c}$ has an acetonato structure include compounds ZR-1 and ZR-3 described later.

In addition, in General Formula (e), it is preferable that at least one of $OR^{2c}$'s has an acetato structure. The acetato structure has the same definition as the acetato structure described by General Formula (c). Examples of the form in which $OR^{2c}$ has an acetato structure include a compound ZR-4 described later.

In addition, in General Formulae (e) and (f), it is preferable that at least one of $OR^{2c}$'s has a lactato structure. The lactato structure means a structure in which a lactic acid ion (lactate) is taken as a basic structure, and one hydrogen ion is removed from the basic structure, and the structure is coordinated to Zr. The phrase "lactic acid ion is taken as a basic structure" means to include, in addition to the lactic acid ion, a structure in which a hydrogen atom of the lactic acid ion is substituted with a substituent. A coordinating atom coordinated to the Zr is usually an oxygen atom. Examples of the form in which $OR^{2c}$ has a lactato structure include a compound ZR-2 described later.

Hereinafter, specific examples of the zirconium alkoxide compound used in the present invention will be given, but the present invention is not limited thereto.

Tetrapropoxyzirconium (also known as zirconium tetra-n-propoxide)
Tetrabutoxyzirconium (also known as zirconium tetra-n-butoxide)
Zirconium tetraacetylacetonate
Zirconium tributoxy monoacetylacetonate
Zirconium dibutoxy bis(acetyl acetonate)
Zirconium dibutoxy bis(ethyl acetoacetate)
Zirconium tributoxyethylacetoacetate
Zirconium monobutoxyacetylacetonate bis(ethyl acetoacetate)
Zirconium tributoxy monostearate (also known as zirconium stearate tri-n-butoxide)
Zirconium stearate
Zirconium lactate ammonium salt
Zirconium monoacetylacetonate

Molecular weights of the silane coupling agent, the titanium alkoxide compound, the aluminum alkoxide compound, and the zirconium alkoxide compound used in the present invention are not particularly limited, and are each, for example, preferably 100 to 2,000 and more preferably 200 to 500.

A polymeric silane coupling agent can also be used.

The silane coupling agent, the aluminum alkoxide compound, the zirconium alkoxide compound, and the titanium alkoxide compound, which can be contained in the interlayer, may be used alone or in combination of two or more kinds thereof.

In a case where the interlayer contains a constitutional component derived from at least one of the silane coupling agent, the titanium alkoxide compound, the aluminum alkoxide compound, or the zirconium alkoxide compound, the total content of the constitutional components derived from the silane coupling agent, the titanium alkoxide compound, the aluminum alkoxide compound, and the zirconium alkoxide compound contained in the interlayer is not particularly limited, and it is preferably 1% by mass or more, more preferably 5% by mass or more, still more preferably 10% by mass or more,

and particularly preferably 15% by mass or more. The upper limit value thereof is not particularly limited, and is, for example, preferably 99% by mass or less, more preferably 95% by mass or less, and still more preferably 90% by mass or less. That is, the above-described total content of the constitutional components derived from the silane coupling agent, the titanium alkoxide compound, the aluminum alkoxide compound, and the zirconium alkoxide compound contained in the interlayer is preferably 1% to 99% by mass, more preferably 5% to 99% by mass, still more preferably 10% to 95% by mass, and particularly preferably 15% to 90% by mass.

The total content of the silane coupling agent, the titanium alkoxide compound, the aluminum alkoxide compound, and the zirconium alkoxide compound in the liquid (containing a solvent) used for forming the interlayer is, for example, preferably 0.02% by mass or more, more preferably 0.1% by mass or more, still more preferably 0.3% by mass or more, and particularly preferably 0.5% by mass or more. The upper limit value thereof is not particularly limited, and is, for example, 99% by mass or less, preferably 70% by mass or less, more preferably 50% by mass or less, and still more preferably 25% by mass or less. That is, the above-described total content of the silane coupling agent, the titanium alkoxide compound, the aluminum alkoxide compound, and the zirconium alkoxide compound in the liquid (containing a solvent) used for forming the interlayer is preferably 0.02% to 99% by mass, more preferably 0.1% to 70% by mass, still more preferably 0.3% to 50% by mass, and particularly preferably 0.5% to 25% by mass.

In the present invention, the expression "the interlayer includes a constitutional component derived from at least one of the silane coupling agent, the titanium alkoxide compound, the aluminum alkoxide compound, or the zirconium alkoxide compound" means that at least one of the silane coupling agent, the titanium alkoxide compound, the aluminum alkoxide compound, or the zirconium alkoxide compound is included in a state of reacting with the acoustic lens layer and/or the substrate. For example, at least a part of the silane coupling agent, the titanium alkoxide compound, the aluminum alkoxide compound, are the zirconium alkoxide compound is hydrolyzed to expose a hydroxy group, and the exposed hydroxy group can be present by reacting with a group on the surface of the acoustic lens layer and/or the substrate.

As long as the effects of the present invention are not impaired, the interlayer may include a component other than the siloxane compound having a constitutional component derived from a hydrolyzed silicate compound and the constitutional component derived from at least one of the silane coupling agent, the titanium alkoxide compound, the aluminum alkoxide compound, or the zirconium alkoxide compound; and examples of such a component include additives such as a surfactant, an antioxidant, a thickener, a leveling agent, a stabilizer, and an antifoaming agent.

The interlayer may be a single layer or a multilayer, and is preferably a single layer.

An average layer thickness of the interlayer is not particularly limited, and is, for example, preferably 5 nm to 100 μm, more preferably 10 nm to 20 μm, still more preferably 25 nm to 10 μm, and particularly preferably 40 nm to 5 μm. The average layer thickness of the interlayer is a value measured by a method described in Examples later.

<Acoustic lens layer>

The acoustic lens layer in the acoustic wave probe according to the embodiment of the present invention is a layer obtained by curing room temperature-curable silicone, and the room temperature curable silicone includes a constitutional unit having a phenyl group.

As described above, since the acoustic lens layer in the acoustic wave probe according to the embodiment of the present invention is a layer obtained by curing room temperature-curable silicone which has a constitutional unit having a phenyl group, it is considered that permeability of the hydrogen peroxide gas and the like in the acoustic lens layer is suppressed, the amount of hydrogen peroxide gas and the like reaching the interface between the acoustic lens layer and the interlayer and the interface between the interlayer and the substrate is reduced, and as a result, excellent hydrogen peroxide plasma sterilization resistance can be exhibited.

A content of the phenyl group in a cured substance of the room temperature-curable silicone is preferably 1 to 50 mol%, more preferably 2 to 30 mol%, and from the viewpoint of further improving the heat cycle resistance and the hydrogen peroxide plasma sterilization resistance, it is still more preferably 3 to 20 mol% and particularly preferably 4 to 10 mol%.

The phenyl group in the cured substance of the room temperature-curable silicone may be unsubstituted or may have a substituent. Examples of the phenyl group having a substituent include an alkyl-substituted phenyl group (group in which a hydrogen atom in a phenyl group is substituted with a linear, branched, or cyclic alkyl group having 1 to 10 carbon atoms), and a group in which a hydrogen atom in a phenyl group is substituted with at least one substituent such as a halogen atom, a hydroxy group, an alkoxy group, an alkoxyalkyl group, an alkoxycarbonyl group, an alkoxycarbonyloxy group, a carboxy group, a cyano group, and a nitro group.

Here, the content of the phenyl group in the cured substance of the room temperature-curable silicone is mol% of a phenyl group-containing siloxane unit in a case where all units constituting the cured substance of the room temperature-curable silicone are set to 100 mol%. One phenyl group-containing siloxane unit has one to three phenyl groups. Among these, the number of phenyl groups is preferably two for one phenyl group-containing siloxane unit. For example, in a case

where all Si atoms of an Si-O unit and a terminal Si have at least one phenyl group, the content is 100 mol%.

The unit refers to the Si-O unit and the terminal Si.

The room temperature-curable silicone is also referred to as RTV silicone, and means silicone that can be cured under a temperature condition of 50°C or lower. For example, a silicone composition containing vinyl silicone and hydrosilicone can be used as the RTV silicone, and can be cured under a temperature condition of 50°C or lower in the presence of a platinum catalyst, and thus the silicone composition can be preferably used.

(Silicone composition)

The silicone composition containing vinyl silicone and hydrosilicone (in the present invention, also simply referred to as "silicone composition") as the room temperature-curable silicone contains vinyl silicone which is polysiloxane having a vinyl group, and hydrosilicone which is polysiloxane having two or more Si-H groups in a molecular chain.

The constitutional unit having a phenyl group may be included as a constitutional unit of at least one of vinyl silicone or hydrosilicone, and it is preferably included as a constitutional unit of at least vinyl silicone.

A content of the vinyl silicone in 100 parts by mass of the total of the silicone composition is preferably 80 to 99.9 parts by mass, and a content of the hydrosilicone is preferably 0.1 to 20 parts by mass. In a case where the silicone composition contains a component other than the vinyl silicone and the hydrosilicone, the content of the vinyl silicone is more preferably 40 to 70 parts by mass and still more preferably 45 to 65 parts by mass. The content of the hydrosilicone is more preferably 0.3 to 15 parts by mass, still more preferably 0.3 to 10 parts by mass, particularly preferably 0.4 to 5 parts by mass, and among these, preferably 0.4 to 3 parts by mass.

From the viewpoint of further suppressing the permeability of the hydrogen peroxide gas and the like in the acoustic lens layer and further improving the hydrogen peroxide plasma sterilization resistance, the above-described silicone composition preferably contains surface-treated oxide particles described later, and more preferably contains surface-treated spherical silica particles described later.

In a case where the silicone composition contains the surface-treated oxide particles, a preferred content of each component is as follows.

The content of the vinyl silicone in 100 parts by mass of the total of the silicone composition is preferably 29.9 to 74.9 parts by mass, and the content of the hydrosilicone is preferably 0.1 to 20 parts by mass. The content of the vinyl silicone is more preferably 40 to 70 parts by mass and still more preferably 45 to 65 parts by mass. The content of the hydrosilicone is more preferably 0.3 to 15 parts by mass, still more preferably 0.3 to 10 parts by mass, particularly preferably 0.4 to 5 parts by mass, and among these, preferably 0.4 to 3 parts by mass.

The content of the surface-treated oxide particles (preferably, the surface-treated spherical silica particles) in 100 parts by mass of the total of the silicone composition is preferably 25 to 70 parts by mass, more preferably 30 to 60 parts by mass, and still more preferably 35 to 50 parts by mass. In a case where the content of the surface-treated oxide particles (preferably, the surface-treated spherical silica particles) is within the above-described range, tear strength, bending durability, and acoustic wave sensitivity are increased.

**In** a case where the content of the polysiloxane (vinyl silicone and hydrosilicone) is within the above-described range, hardness, tear strength, and acoustic impedance of the obtained cured substance (silicone resin) are excellent in balance.

The silicone composition is a composition which does not contain a catalyst for crosslinking and polymerizing (curing) the vinyl silicone and the hydrosilicone. Therefore, the silicone composition contains silica particles, but does not contain the catalyst.

**In** addition, 100 parts by mass of the total of the silicone composition means that the total of individual components contained in the silicone composition is 100 parts by mass.

Any silicone may be used as the above-described silicone contained in the silicone composition, as long as the silicone has a vinyl group or has two or more Si-H groups in the molecular chain. However, in the present invention, the vinyl silicone is preferably the following polyorganosiloxane (A) having a vinyl group, and the hydrosilicone is preferably the following polyorganosiloxane (B) having two or more Si-H groups in the molecular chain.

The constitutional unit having a phenyl group may be included as at least one constitutional unit of the polyorganosiloxane (A) having a vinyl group or the polyorganosiloxane (B) having two or more Si-H groups in the molecular chain, and it is preferably included as at least a constitutional unit of the polyorganosiloxane (A) having a vinyl group.

Therefore, as the silicone composition in the present invention, a composition which contains, as components, at least the polyorganosiloxane (A) having a vinyl group and the polyorganosiloxane (B) having two or more Si-H groups in the molecular chain is preferable, and a composition which contains, as components, at least the polyorganosiloxane (A) having a vinyl group, the polyorganosiloxane (B) having two or more Si-H groups in the molecular chain, and silica particles (C) is more preferable.

In the following detailed description, a silicone composition containing the polyorganosiloxane (A) having a vinyl

group and the polyorganosiloxane (B) having two or more Si-H groups in the molecular chain, which is the preferred aspect, will be described. However, each silicone contained in the silicone composition is not limited to the polyorganosiloxanes (A) and (B).

(Polyorganosiloxane (A) having vinyl group)

The polyorganosiloxane (A) having a vinyl group (hereinafter, also simply referred to as a polyorganosiloxane (A)) used in the present invention preferably has two or more vinyl groups in the molecular chain.

Examples of the polyorganosiloxane (A) include a polyorganosiloxane (a) having vinyl groups at least at both terminals of the molecular chain (hereinafter, also simply referred to as a polyorganosiloxane (a)) and a polyorganosiloxane (b) having at least two $-O-Si(CH_3)_2(CH=CH_2)$ in the molecular chain (hereinafter, also simply referred to as a polyorganosiloxane (b)). Among these, the polyorganosiloxane (a) having vinyl groups at least at both terminals of the molecular chain is preferable.

The polyorganosiloxane (a) is preferably linear; and in the polyorganosiloxane (b), it is preferable that $-O-Si(CH_3)_2(CH=CH_2)$ is bonded to the Si atom constituting the main chain.

The polyorganosiloxane (A) is, for example, hydrosilylated by reacting with a polyorganosiloxane (B) having two or more Si-H groups in the presence of a platinum catalyst. A crosslinking structure (cured substance) can be formed by the hydrosilylation reaction (addition reaction).

A content of the vinyl group in the polyorganosiloxane (A) is not particularly limited. From the viewpoint of forming a sufficient network with each component contained in the acoustic lens layer, the content of the vinyl group is, for example, preferably 0.01 to 5 mol% and more preferably 0.05 to 2 mol%.

Here, the content of the vinyl group is mol% of a vinyl group-containing siloxane unit in a case where all units constituting the polyorganosiloxane (A) are set to 100 mol%. One vinyl group-containing siloxane unit has one to three vinyl groups. Among these, the number of vinyl groups is preferably one for one vinyl group-containing siloxane unit. For example, in a case where all Si atoms of an Si-O unit and a terminal Si constituting the main chain have at least one vinyl group, the content is 100 mol%.

In addition, the polyorganosiloxane (A) preferably has a phenyl group. From the viewpoint of mechanical strength and hydrogen peroxide plasma sterilization resistance in a case of being formed into the acoustic lens layer, a content of the phenyl group is, for example, preferably 1 to 50 mol%, more preferably 2 to 30 mol%, and still more preferably 3 to 20 mol%, and from the viewpoint of further improving the heat cycle resistance and the hydrogen peroxide plasma sterilization resistance, it is particularly preferably 4 to 10 mol%.

Here, the content of the phenyl group is mol% of a phenyl group-containing siloxane unit in a case where all units constituting the polyorganosiloxane (A) are set to 100 mol%. One phenyl group-containing siloxane unit has one to three phenyl groups. Among these, the number of phenyl groups is preferably two for one phenyl group-containing siloxane unit. For example, in a case where all Si atoms of an Si-O unit and a terminal Si constituting the main chain have at least one phenyl group, the content is 100 mol%.

The unit refers to the Si-O unit and the terminal Si constituting the main chain.

A degree of polymerization and specific gravity are not particularly limited. From the viewpoint of improving the mechanical strength, hardness, chemical stability, and the like of the silicone resin constituting the acoustic lens layer to be obtained (hereinafter, also simply referred to as a silicone resin), the degree of polymerization is preferably 200 to 3,000 and more preferably 400 to 2,000, and the specific gravity is preferably 0.9 to 1.1.

From the viewpoint of mechanical strength, hardness, and/or ease of processing, a mass-average molecular weight of the polyorganosiloxane (A) is preferably 20,000 to 200,000, more preferably 40,000 to 150,000, and still more preferably 45,000 to 120,000.

In the present invention, the mass-average molecular weight can be measured, for example, by using a GPC device HLC-8220 (trade name, manufactured by Tosoh Corporation), toluene (manufactured by FUJIFILM Wako Pure Chemical Corporation) as an eluent, TSKgel (registered trademark) G3000HXL + TSKgel (registered trademark) G2000HXL as columns, and a differential refractive index (RI) detector under the conditions of a temperature of 23°C and a flow rate of 1 mL/min.

A kinematic viscosity of the polyorganosiloxane (A) at 25°C is preferably $1 \times 10^{-5}$ to 10 $m^2/s$, more preferably $1 \times 10^{-4}$ to 1 $m^2/s$, and still more preferably $1 \times 10^{-3}$ to 0.5 $m^2/s$.

The kinematic viscosity can be determined by measuring at a temperature of 25°C using a Ubbelohde type viscometer (for example, manufactured by Sibata Scientific Technology Ltd., trade name: SU) in accordance with JIS Z 8803.

The polyorganosiloxane (a) having vinyl groups at least at both terminals of the molecular chain is preferably a polyorganosiloxane represented by General Formula (A).

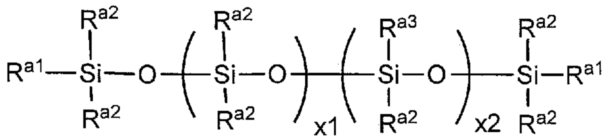 (A)

In General Formula (A), $R^{a1}$ represents a vinyl group, and $R^{a2}$ and $R^{a3}$ each independently represent an alkyl group, a cycloalkyl group, an alkenyl group, or an aryl group. x1 and x2 each independently represent an integer of 1 or more.

The number of carbon atoms in the alkyl group in $R^{a2}$ and $R^{a3}$ is preferably 1 to 10, more preferably 1 to 4, still more preferably 1 or 2, and particularly preferably 1. Examples of the alkyl group include methyl, ethyl, n-propyl, isopropyl, n-butyl, isobutyl, n-hexyl, n-octyl, 2-ethylhexyl, and n-decyl.

The number of carbon atoms in the cycloalkyl group in $R^{a2}$ and $R^{a3}$ is preferably 3 to 10, more preferably 5 to 10, and still more preferably 5 or 6. In addition, the cycloalkyl group is preferably a 3-membered ring, a 5-membered ring, or a 6-membered ring, and more preferably a 5-membered ring or a 6-membered ring. Examples of the cycloalkyl group include cyclopropyl, cyclopentyl, and cyclohexyl.

The number of carbon atoms in the alkenyl group in $R^{a2}$ and $R^{a3}$ is preferably 2 to 10, more preferably 2 to 4, and still more preferably 2. Examples of the alkenyl group include vinyl, allyl, and butenyl.

The number of carbon atoms in the aryl group in $R^{a2}$ and $R^{a3}$ is preferably 6 to 12, more preferably 6 to 10, and still more preferably 6 to 8. Examples of the aryl group include phenyl, tolyl, and naphthyl.

The alkyl group, the cycloalkyl group, the alkenyl group, and the aryl group each may have a substituent. Examples of such a substituent include a halogen atom, an alkyl group, a cycloalkyl group, an alkenyl group, an aryl group, an alkoxy group, an aryloxy group, an alkylthio group, an arylthio group, a silyl group, and a cyano group.

Examples of the group having a substituent include a halogenated alkyl group.

$R^{a2}$ and $R^{a3}$ are preferably an alkyl group, an alkenyl group, or an aryl group, more preferably an alkyl group having 1 to 4 carbon atoms, a vinyl group, or a phenyl group, still more preferably a methyl group, a vinyl group, or a phenyl group, and particularly preferably a methyl group or a phenyl group.

Among these, in a case where the polyorganosiloxane (a) having vinyl groups at least at both terminals of the molecular chain includes a constitutional unit having a phenyl group, it is preferable that both $R^{a2}$ and $R^{a3}$ in the repeating unit represented by $()_{x2}$ are a phenyl group, and $R^{a2}$ at other portions is a methyl group, that is, the polyorganosiloxane (a) is preferably a vinyl-terminated diphenylsiloxane-dimethylsiloxane copolymer.

x1 is preferably an integer of 200 to 3,000 and more preferably an integer of 400 to 2,000.

x2 is preferably an integer of 1 to 3,000, more preferably an integer of 1 to 1,000, still more preferably an integer of 40 to 1,000, and particularly preferably an integer of 40 to 700.

In addition, as another aspect, x1 is preferably an integer of 1 to 3,000 and more preferably an integer of 5 to 1,000.

Examples of the polyorganosiloxane having vinyl groups at least at both terminals of a molecular chain thereof include DMS series (for example, DMS-V31, DMS-V31S15, DMS-V33, DMS-V35, DMS-V35R, DMS-V41, DMS-V42, DMS-V46, DMS-V51, and DMS-V52), PDV series (for example, PDV-0341, PDV-0346, PDV-0535, PDV-0541, PDV-1631, PDV-1635, PDV-1641, and PDV-2335), PMV-9925, PVV-3522, FMV-4031, and EDV-2022, all of which are trade names manufactured by Gelest, Inc.

The DMS-V31S15 is pre-formulated with fumed silica, so that no kneading with a special device is required.

The polyorganosiloxane (A) having a vinyl group in the present invention may be used alone, or in combination of two or more kinds thereof.

(Polyorganosiloxane (B) having two or more Si-H groups in molecular chain)

The polyorganosiloxane (B) having two or more Si-H groups in the molecular chain (hereinafter, also simply referred to as a polyorganosiloxane (B)) used in the present invention has two or more Si-H groups in the molecular chain. Here, in a case where the component (b) has a "$-SiH_2-$" structure, the number of Si-H groups in the "$-SiH_2-$" structure is counted as two. In addition, in a case where the component (b) has a "$-SiH_3$" structure, the number of Si-H groups in the "$-SiH_3$" structure is counted as three.

In a case of having two or more Si-H groups in the molecular chain, a crosslinking structure can be formed by reacting with a polyorganosiloxane having a polymerizable unsaturated group (a vinyl group or the like) (preferably, a polyorganosiloxane having at least two polymerizable unsaturated groups (a vinyl group or the like)).

The polyorganosiloxane (B) has a linear structure or a branched structure, and a linear structure is preferable.

From the viewpoint of mechanical strength and hardness of the acoustic lens layer, a mass-average molecular weight of the linear structure is preferably 500 to 100,000 and more preferably 1,500 to 50,000.

The polyorganosiloxane (B) having a linear structure and having two or more Si-H groups in the molecular chain is preferably a polyorganosiloxane represented by General Formula (B).

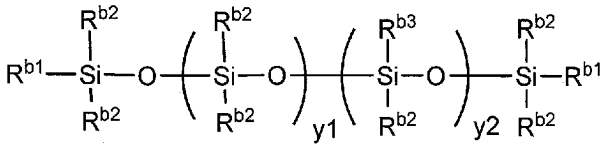 (B)

In General Formula (B), $R^{b1}$ to $R^{b3}$ each independently represent a hydrogen atom, an alkyl group, a cycloalkyl group, an alkenyl group, an aryl group, or $-O-Si(R^{b5})_2(R^{b4})$. $R^{b4}$ and $R^{b5}$ each independently represent a hydrogen atom, an alkyl group, a cycloalkyl group, an alkenyl group, or an aryl group. y1 represents an integer of 0 or more, and y2 represents an integer of 1 or more. Here, a plurality of $R^{b1}$'s, a plurality of $R^{b2}$'s, a plurality of $R^{b3}$'s, a plurality of $R^{b4}$'s, and a plurality of $R^{b5}$'s may be the same or different from each other; and each of the groups of $R^{b1}$ to $R^{b5}$ may be further substituted with a substituent. Here, the molecular chain has two or more Si-H groups.

The alkyl group, the cycloalkyl group, the alkenyl group, and the aryl group in $R^{b1}$ to $R^{b3}$ have the same meanings as the alkyl group, the cycloalkyl group, the alkenyl group, and the aryl group in $R^{a2}$ and $R^{a3}$, and preferred aspects thereof are also the same.

The alkyl group, the cycloalkyl group, the alkenyl group, and the aryl group in $R^{b4}$ and $R^{b5}$ of $-O-Si(R^{b5})_2(R^{b4})$ have the same meanings as the alkyl group, the cycloalkyl group, the alkenyl group, and the aryl group in $R^{b1}$ to $R^{b3}$, and preferred aspects thereof are also the same.

$R^{b1}$ to $R^{b3}$ are preferably a hydrogen atom, an alkyl group, an alkenyl group, an aryl group, or $-O-Si(R^{b5})_2$ $(R^{b4})$, and more preferably a hydrogen atom, an alkyl group having 1 to 4 carbon atoms, a vinyl group, a phenyl group, or $-O-Si(CH_3)_2H$.

Among these, $R^{b1}$ and $R^{b2}$ are more preferably a hydrogen atom, an alkyl group, an alkenyl group, or an aryl group, still more preferably a hydrogen atom or an alkyl group, and particularly preferably a hydrogen atom or a methyl group.

$R^{b3}$ is more preferably a hydrogen atom or an aryl group, still more preferably a hydrogen atom or a phenyl group, and particularly preferably a hydrogen atom.

In the present invention, in a case where $R^{b3}$ is a phenyl group, $R^{b1}$ is preferably a hydrogen atom, and it is more preferable that $R^{b1}$ is a hydrogen atom and the following conditions are satisfied.

1) one $R^{b2}$ in repetition of y1's is a hydrogen atom, the remaining $R^{b2}$'s are alkyl groups, $R^{b2}$'s in repetition of y2's are alkyl groups, and $R^{b3}$ is a phenyl group.
2) y1 is 0, $R^{b2}$ in repetition of y2's is an alkyl group, and $R^{b3}$ is a phenyl group.
3) y1 is 0, $R^{b2}$ in repetition of y2's is $-O-Si(R^{b5})_2(R^{b4})$, and $R^{b3}$ is a phenyl group.

In the above 3), a case where $R^{b4}$ is a hydrogen atom and $R^{b5}$ is an alkyl group is particularly preferable.

y1 is preferably an integer of 0 to 2,000, more preferably an integer of 0 to 1,000, and still more preferably an integer of 0 to 30.

y2 is preferably an integer of 1 to 2,000, more preferably an integer of 1 to 1,000, and still more preferably an integer of 1 to 30.

y1 + y2 is preferably an integer of 5 to 2,000, more preferably an integer of 7 to 1,000, still more preferably an integer of 10 to 50, and even still more preferably an integer of 15 to 30.

In the present invention, "$-Si(R^{b2})_2-O-$" and "$-Si(R^{b2})(R^{b3})_2-O-$" in General Formula (B) may each exist in a block-polymerized form in polysiloxane or may be in a form that exists at random.

A combination of $R^{b1}$ to $R^{b3}$ is preferably a combination of $R^{b1}$ being a hydrogen atom or an alkyl group having 1 to 4 carbon atoms, $R^{b2}$ being a hydrogen atom or an alkyl group having 1 to 4 carbon atoms, and $R^{b3}$ being a hydrogen atom or an aryl group; and more preferably a combination of $R^{b1}$ being an alkyl group having 1 to 4 carbon atoms, $R^{b2}$ being an alkyl group having 1 to 4 carbon atoms, and $R^{b3}$ being a hydrogen atom.

In this preferred combination, the content of the hydrosilyl group represented by y2/(y1 + y2) is preferably more than 0.1 and 1.0 or less, and more preferably more than 0.2 and 1.0 or less. That is, the form includes a form in which y1 is 0.

Examples of the polyorganosiloxane (B) having a linear structure include HMS-064 (MeHSiO: 5 to 7 mol%), HMS-082 (MeHSiO: 7 to 8 mol%), HMS-301 (MeHSiO: 25 to 30 mol%), and HMS-501 (MeHSiO: 50 to 55 mol%) as methylhydrosiloxane-dimethylsiloxane copolymers (trimethylsiloxane terminated), HPM-502 (MeHSiO: 45 to 50 mol%) as a methylhydrosiloxane-phenylmethylsiloxane copolymer, and HMS-991 (MeHSiO: 100 mol%) as a methylhydrosiloxane polymer, all of which are manufactured by Gelest, Inc.

Here, the mol% of MeHSiO has the same meaning as that y2/(y1 + y2) in the preferred combination of $R^{b1}$ to $R^{b3}$ is

multiplied by 100.

From the viewpoint of preventing the progress of the crosslinking reaction in the molecule, it is preferable that both the linear structure and the branched structure do not have a vinyl group, and among these, it is preferable that the branched structure does not have a vinyl group.

As the polyorganosiloxane (B) having a branched structure and having two or more Si-H groups in the molecular chain, the description of a polyorganosiloxane having a branched structure described in [0051] to [0058] of WO2017/130890A can be applied as it is.

The polyorganosiloxane (B) having two or more Si-H groups in the molecular chain used in the present invention may be used alone, or in combination of two or more kinds thereof. In addition, the polyorganosiloxane (B) having a linear structure and the polyorganosiloxane (B) having a branched structure may be used in combination.

The vinyl group included in the polyorganosiloxane (A) and the Si-H group included in the polyorganosiloxane (B) are usually reacted in a stoichiometric ratio of 1:1.

However, from the viewpoint that all the vinyl groups react with the Si-H group, the equivalent of the Si-H group included in the polyorganosiloxane (B) with respect to the vinyl group included in the polyorganosiloxane (A) is preferably in a range of vinyl group:Si-H group = 1:1.1 to 1:8 and more preferably in a range of vinyl group:Si-H group = 1:1.2 to 1:5.

(Surface-treated oxide particles)

As described above, the above-described silicone composition preferably contains surface-treated oxide particles.

The surface-treated oxide particles mean particles in which a surface of inorganic oxide particles is surface-treated.

From the viewpoint of improving the acoustic impedance, hardness, and mechanical strength of the acoustic lens layer, an average primary particle diameter of the surface-treated oxide particles is preferably more than 3 nm and less than 500 nm, more preferably more than 16 nm and less than 250 nm, still more preferably 18 to 100 nm, particularly preferably 20 to 80 nm, and most preferably 25 to 70 nm.

By adding the surface-treated oxide particles to the silicone composition, an effect of improving the acoustic impedance, hardness, and mechanical strength of the acoustic lens layer to be obtained can be obtained.

In particular, in a case where the surface-treated oxide particles having an average primary particle diameter of more than 16 nm and less than 250 nm are used, it is possible to reduce the attenuation amount of acoustic wave and reduce the viscosity before curing.

Here, the average primary particle diameter refers to a volume average particle size. The volume average particle size can be calculated, for example, by measuring a particle size distribution using a laser diffraction scattering-type particle size distribution analyzer (for example, manufactured by HORIBA, Ltd., trade name "LA910"). In the present specification, the average primary particle diameter obtained by the above-described measuring method is an average primary particle diameter of a product for which the average primary particle diameter is not described in the catalog or which is newly manufactured.

Here, the average primary particle diameter of the surface-treated oxide particles means an average primary particle diameter in a surface-treated state.

In addition, as the inorganic oxide particles constituting the surface-treated oxide particles, particles of an inorganic oxide selected from silica, titanium oxide, aluminum oxide, zinc oxide, zirconium oxide, magnesium oxide, and calcium oxide are preferable; particles of an inorganic oxide selected from silica, titanium oxide, and zinc oxide are more preferable; and silica particles are still more preferable.

The surface-treated oxide particles may be used alone or in combination of two or more thereof.

From the viewpoint of improving the hardness and/or mechanical strength of the silicone resin to be obtained, a specific surface area of the surface-treated oxide particles used in the present invention is preferably 1 to 400 $m^2/g$, more preferably 5 to 200 $m^2/g$, and particularly preferably 10 to 100 $m^2/g$.

Examples of the surface treatment method for the surface-treated oxide particles used in the present invention include a surface treatment with an inorganic compound and/or an organic compound; and a surface treatment with a silane compound is preferable.

For example, an inorganic surface treatment with aluminum hydroxide, aluminum oxide, zirconium oxide, silica, and/or cerium oxide can be performed.

Specific examples of an organic surface treatment agent which can be used for the surface treatment include silicone oils such as dimethylpolysiloxane, methylhydrogenpolysiloxane, a (dimethicone/methicone) copolymer, methylphenylsilicone, amino-modified silicone, triethoxysilylethylpolydimethylsiloxyethyl dimethicone, and triethoxysilylethylpolydimethylsiloxyethyl hexyl dimethicone; alkyl silanes such as caprylyl silane, decyl silane, and perfluorooctyl silane; alkyl titanate, alkyl aluminate, polyolefin, polyester, amino acids such as lauroyl lysine, polyamide, and salts thereof; metal soaps such as aluminum stearate, aluminum isostearate, and zinc stearate; and water-repellent resins such as nylon,

polyester, and polyacryl. In addition, a coupling agent such as a silane coupling agent and a titanium coupling agent can also be used. The surface treatment can be performed with at least one compound selected from these organic compounds.

In addition, by additionally performing a surface treatment, it is also possible to perform the surface treatment by combining the inorganic surface treatment and the organic surface treatment. In particular, in order to improve the dispersibility in the composition, it is very effective to perform the organic surface treatment.

Examples of the method of the surface treatment with a silane compound include a method of performing the surface treatment with a silane coupling agent and a method of coating with a silicone compound.

(i) Silane coupling agent

From the viewpoint of improving the hardness and/or mechanical strength of the acoustic lens layer, a silane coupling agent having a hydrolyzable group is preferable as the silane coupling agent. The hydrolyzable group in the silane coupling agent is hydrolyzed by water to form a hydroxyl group, and this hydroxyl group undergoes a dehydration condensation reaction with a hydroxyl group on the surface of the silica particles, so that the surface of the silica particles is modified and the hardness and/or mechanical strength of the obtained silicone resin are improved. Examples of the hydrolyzable group include an alkoxy group, an acyloxy group, and a halogen atom.

In a case where the surface of the surface-treated oxide particles is hydrophobically surface-modified, affinity between the surface-treated oxide particles and the polyorganosiloxanes (A) and (B) is improved, and the hydrogen peroxide plasma sterilization resistance is improved, which is preferable.

Examples of the silane coupling agent having an alkyl group or a hydrophobic group such as an aryl group as a reactive functional group include alkoxysilanes such as methyltrimethoxysilane (MTMS), dimethyldimethoxysilane, phenyltrimethoxysilane, methyltriethoxysilane, dimethyl diethoxysilane, phenyltriethoxysilane, n-propyltrimethoxysilane, n-propyltriethoxysilane, hexyltrimethoxysilane, hexyltriethoxysilane, and decyltrimethoxysilane; chlorosilanes such as methyltrichlorosilane, dimethyl dichlorosilane, trimethylchlorosilane, and phenyltrichlorosilane; and hexamethyldisilazane (HMDS).

In addition, examples of the silane coupling agent having a vinyl group as a reactive functional group include alkoxysilanes such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyl trimethoxysilane, and vinylmethyldimethoxysilane; chlorosilanes such as vinyltrichlorosilane and vinylmethyldichlorosilane; and divinyltetramethyldisilazane.

The surface-treated oxide particles which have been subjected to the surface treatment with the silane coupling agent are preferably surface-treated oxide particles which have been treated with a trialkylsilylating agent, and more preferably surface-treated oxide particles which have been treated with a trimethylsilylating agent.

Examples of the silane compound include the above-described silane coupling agent and a silane coupling agent in which a part of the alkoxy group in the above-described silane coupling agent is replaced with an alkyl group.

In addition, examples of the trimethylsilylating agent include trimethylchlorosilane, hexamethyldisilazane (HMDS), and methyltrimethoxysilane (MTMS) described in the above-described silane coupling agent; and trimethylmethoxysilane which is a silane coupling agent in which a part of the alkoxy group in the above-described silane coupling agent is replaced with an alkyl group.

Examples of a commercially available silane coupling agent include hexamethyldisilazane (HMDS) (for example, trade name: HEXAMETHYLDISILAZANE (SIH6110.1), available from Gelest, Inc.).

The hydroxyl group present on the surface of the oxide particles is reacted with hexamethyldisilazane (HMDS), methyltrimethoxysilane (MTMS), trimethylmethoxysilane, or the like to be covered with a trimethylsilyl group, and the surface of the silica particles is reformed to be hydrophobic.

In the present invention, the silane coupling agent may be used alone or in combination of two or more kinds thereof.

(ii) Silicone compound

The silicone compound coating the surface-treated oxide particles may be any polymer composed of a siloxane bond.

Examples of the silicone compound include a silicone compound in which all or some of side chains and/or terminals of polysiloxane are methyl groups, a silicone compound in which some of side chains are hydrogen atoms, a modified silicone compound in which organic groups such as an amino group and/or an epoxy group are introduced into all or some of side chains and/or terminals, and a silicone resin having a branched structure. The silicone compound may have a linear structure or a cyclic structure.

Examples of the silicone compound in which all or some of side chains and/or terminals of polysiloxane are

methyl groups include monomethyl polysiloxanes such as polymethylhydroxysiloxane (hydrogen-terminated), polymethylhydroxysiloxane (trimethylsiloxy-terminated), polymethylphenylsiloxane (hydrogen-terminated), and polymethylphenylsiloxane (trimethylsiloxy-terminated); and dimethyl polysiloxanes such as dimethyl polysiloxane (hydrogen-terminated), dimethyl polysiloxane (trimethylsiloxy-terminated), and cyclic dimethyl polysiloxane.

Examples of the silicone compound in which a part of a side chain is a hydrogen atom include a methylhydrosiloxane-dimethylsiloxane copolymer (trimethylsiloxy-terminated), a methylhydrosiloxane-dimethylsiloxane copolymer (hydrogen-terminated), polymethylhydrosiloxane (hydrogen-terminated), polymethylhydrosiloxane (trimethylsiloxy-terminated), polyethylhydrosiloxane (triethylsiloxy-terminated), polyphenyl-(dimethylhydrosyloxy)siloxane (hydrogen-terminated), a methylhydrosiloxane-phenylmethylsiloxane copolymer (hydrogen-terminated), a methylhydrosiloxane-octylmethylsiloxane copolymer, and a methylhydrosiloxane-octylmethylsiloxane-dimethylsiloxane terpolymer.

In addition, examples of the modified silicone into which an organic group is introduced include reactive silicones into which an organic group such as an amino group, an epoxy group, a methoxy group, a (meth)acryloyl group, a phenoxy group, a carboxylic acid anhydride group, a hydroxy group, a mercapto group, a carboxy group, and/or a hydrogen atom is introduced; and non-reactive silicones modified with a polyether, an aralkyl, a fluoroalkyl, a long-chain alkyl, a long-chain aralkyl, a higher fatty acid ester, a higher fatty acid amide, and/or a polyether methoxy.

The silica particles coated with a silicone compound can be obtained by a conventional method. For example, the silica particles coated with a silicone compound are obtained by mixing and stirring the silica particles in dimethyl polysiloxane for a certain period of time and filtering the mixture.

In addition, in a case where a reactive modified silicone is used as the silicone compound, the organic group reacts with the hydroxyl group on the surface of the oxide particles, and the surface of the oxide particles is reformed, and the hardness and/or mechanical strength of the acoustic lens layer to be obtained is improved.

Examples of a commercially available silicone compound include methylhydrogenosilicone oil (MHS) (trade name: KF-99, manufactured by Shin-Etsu Chemical Co., Ltd.) which is polymethylhydrosiloxane (trimethylsiloxy terminal).

A methanol hydrophobicity degree of the surface-treated oxide particles, calculated by the following methanol titration test, is preferably 40% to 80% by mass, more preferably 50% to 80% by mass, and still more preferably 60% to 80% by mass. Here, a larger methanol hydrophobicity degree indicates a higher hydrophobicity, and a smaller methanol hydrophobicity degree indicates a higher hydrophilicity.

50 ml of ion exchange water and 0.2 g of the surface-treated oxide particles as a sample are put into a beaker and the temperature is set to 25°C, and methanol is added dropwise from a burette to the beaker while stirring with a magnetic stirrer, and an amount (X g) of methanol added dropwise until the entire amount of the sample sinks is measured. The methanol hydrophobicity degree is calculated from the following expression.

$$\text{Methanol hydrophobicity degree (\% by mass)} = 100\% \times X/(50 + X)$$

In a case where the methanol hydrophobicity degree is within the above-described preferred range, the viscosity of the silicone composition before curing is not increased, and a decrease in acoustic wave sensitivity in a case of being formed into the acoustic lens layer can be suppressed.

A sphericity of Wadell of primary particles of the surface-treated oxide particles is preferably 0.7 to 1, more preferably 0.8 to 1, and still more preferably 0.9 to 1.

Here, the "sphericity of Wadell" (see Chemical Engineering Handbook, published by Maruzen Co., Ltd.) is an index for measuring sphericity of a particle by (Diameter of circle equal to projected area of particle)/(Diameter of minimum circle circumscribing projected image of particle), and it means that the particle is closer to a true sphere as the index is closer to 1.0.

For the measurement of the sphericity of Wadell (hereinafter, also simply referred to as sphericity), for example, a scanning electron microscope (SEM) image can be used. Specifically, for example, approximately 100 primary particles are observed with the SEM image, and the sphericity of the primary particles is calculated. An average value obtained by dividing the calculated total of sphericity by the number of the observed primary particles is defined as the sphericity.

In a case where the sphericity of Wadell is within the above-described preferred range, the hydrogen peroxide plasma sterilization resistance is further improved, which is preferable; and the area of the acoustic wave incident on the surface-treated oxide particles is considered to be reduced in a case where the acoustic lens layer is irradiated with an acoustic wave, and thus the acoustic wave sensitivity is considered to be improved.

In the present specification, "spherical" includes slightly distorted spheres having a sphericity of Wadell of 0.9 to 1, and the above-described surface-treated spherical silica particles mean spherical particles among the surface-treated silica particles.

In the oxide particles, the silica particles are roughly classified according to the production method thereof into combustion method silica (that is, fumed silica) obtained by burning a silane compound, explosion method silica obtained

by explosively burning metal silicon powder, wet silica obtained by a neutralization reaction of sodium silicate and mineral acid (silica obtained by synthesis under alkaline conditions is called sedimentation method silica and silica obtained by synthesis under acidic conditions is called gel method silica), and sol-gel method silica obtained by hydrolysis of hydrocarbyloxy silane (so-called Stoeber method).

Examples of a method for producing true sphere-shaped spherical silica particles include an explosion method and a sol-gel method, which are preferable.

The sol-gel method is a method of obtaining hydrophilic spherical silica particles essentially consisting of $SiO_2$ units by hydrolyzing and condensing a hydrocarbyloxysilane (preferably, tetrahydrocarbyloxysilane), a partially hydrolyzed and condensed product of the hydrocarbyloxysilane, or a combination thereof.

In addition, the hydrophobic treatment of the surface of the silica particles can be performed by introducing an $R^*_3SiO_{1/2}$ unit ($R^3$'s are the same or different from each other, and each represent a substituted or unsubstituted monovalent hydrocarbon group having 1 to 20 carbon atoms) onto the hydrophilic surface of the spherical silica particles.

Specifically, for example, the hydrophobic treatment can be performed by methods described in JP2007-99582A and JP2014-114175A. The surface treatment of the other oxide particles is not particularly limited, and can be performed by a conventional method.

(Other components)

In addition to the polyorganosiloxane (A) having a vinyl group, the polyorganosiloxane (B) having two or more Si-H groups in the molecular chain, and the surface-treated oxide particles, at least one of a curing retarder, a solvent, a dispersant, a pigment, a dye, an antistatic agent, an antioxidant, a flame retardant, or a thermal conductivity improver can be appropriately blended into the above-described silicone composition.

- Curing retarder -

In the present invention, a curing retarder for the curing reaction can be appropriately used. The curing retarder is used for the purpose of delaying the above-described addition curing reaction, and examples thereof include a low molecular weight vinyl methylsiloxane homopolymer (trade name: VMS-005, manufactured by Gelest, Inc.).

A curing rate, that is, a working time can be adjusted depending on the content of the curing retarder.

- Catalyst -

In the present invention, a catalyst is allowed to act in order to obtain the layer obtained by curing the above-described silicone composition. Examples of the catalyst include platinum or a platinum-containing compound (hereinafter, also simply referred to as a platinum compound). As the platinum or the platinum compound, those used for room temperature curing (RTV) can be used without particular limitation, and for example, platinum-vinyl disiloxane can be used.

Specific examples thereof include platinum black or platinum supported on an inorganic compound or carbon black, chloroplatinic acid or an alcohol solution of chloroplatinic acid, a complex salt of chloroplatinic acid and olefin, and a complex salt of chloroplatinic acid and vinyl siloxane. The catalyst may be used alone or in combination of two or more thereof.

The catalyst is required in a hydrosilylation reaction in which the Si-H group of the polyorganosiloxane (B) is added to the vinyl group of the polyorganosiloxane (A). As the hydrosilylation reaction (addition curing reaction) proceeds, the polyorganosiloxane (A) is crosslinked with the polyorganosiloxane (B) to form the silicone resin.

Here, the catalyst may be allowed to act by being mixed with the above-described silicone composition, or may be allowed to act by being brought into contact with the silicone composition without being contained in the silicone composition. The latter is preferable.

Examples of commercially available platinum catalysts include platinum compounds (trade name: PLATINUM CYCLOVINYLMETHYLSILOXANE COMPLEX IN CYCLIC METHYLVINYLSILOXANES (SIP6832.2), Pt concentration: 2% by mass, and trade name: PLATINUM DIVINYLTETRAMETHYLDISILOXANE COMPLEX IN VINYL-TERMINATED POLYDIMETHYLSILOXANE (SIP6830.3), Pt concentration: 3% by mass; both manufactured by Gelest, Inc.).

A content of the catalyst which acts on the above-described silicone composition is not particularly limited, but from the viewpoint of reactivity, it is preferably 0.00001 to 0.05 parts by mass, more preferably 0.00001 to 0.01 parts by mass, still more preferably 0.00002 to 0.01 parts by mass, and particularly preferably 0.00005 to 0.005 parts by mass with respect to 100 parts by mass of the silicone composition.

[Viscosity of silicone composition before curing]

From the viewpoint of uniformly dispersing each component contained in the silicone composition, it is preferable that the viscosity of the silicone composition before the curing reaction is low. The viscosity before the curing is measured by measuring the viscosity of the silicone composition before adding the catalyst which initiates the curing reaction. Specifically, the viscosity of the composition for an acoustic lens can be measured by the method disclosed in WO2017/130890A.

The above-described viscosity (23°C) is preferably 5,000 Pa·s or less, more preferably 1,000 Pa·s or less, and still more preferably 200 Pa·s or less. The practical lower limit thereof is 10 Pa·s or more.

In the acoustic wave probe according to the embodiment of the present invention, other layers may be further provided between the substrate and the interlayer.

As the other layers, a layer can be used in the acoustic wave probe without particular limitation as long as it has excellent adhesiveness to the substrate and has a reactive group which reacts (preferably forms a chemical bond) with the -OH group included in the hydrolyzed silicate compound in the interlayer. For example, a polyimide layer, which is known as a layer provided to impart resistance to a drug, such as sterilization or disinfection, between the acoustic matching layer and the acoustic lens in the acoustic wave probe, can be preferably used.

In addition, the reactive group which reacts (preferably forms a chemical bond) with the -OH group included in the hydrolyzed silicate compound in the interlayer, which is contained in the other layers, can also be introduced by forming a layer having excellent adhesiveness with the substrate and then subjecting the surface of the layer to a chemical treatment and/or a physical treatment.

<Manufacturing method of acoustic wave probe>

The acoustic wave probe according to the embodiment of the present invention can be prepared by a conventional method.

For example, the substrate, the liquid for forming the interlayer, and the room temperature-curable silicone for forming the acoustic lens layer are prepared by a conventional method, respectively.

In a case where the surface of the substrate on a side where the interlayer is provided is subjected to a hydrophilization treatment, the side where the interlayer is provided is subjected to a hydrophilization treatment by a conventional method.

As the liquid for forming the interlayer, a liquid containing the hydrolyzed silicate compound is prepared by hydrolyzing the silicate compound having a hydrolyzable group, or a liquid is prepared by adding and mixing the commercially available hydrolyzed silicate compound. In a case where the liquid containing the hydrolyzed silicate compound is prepared by hydrolyzing the silicate compound having a hydrolyzable group, it is preferable to perform a neutralization treatment. Other components such as the silane coupling agent, the titanium alkoxide compound, the aluminum alkoxide compound, and the zirconium alkoxide compound, which may be contained in the liquid for forming the interlayer, may be added and mixed at the same time as the preparation of the liquid, or may be separately added and mixed. In a case where the silicate compound having a hydrolyzable group is prepared by hydrolysis, it is preferable that the other components are simultaneously added and mixed after the neutralization treatment.

The room temperature-curable silicone for forming the acoustic lens layer can be obtained, for example, by kneading components constituting the room temperature-curable silicone with a kneader, a pressure kneader, a Banbury mixer (continuous kneader), or a two-roll kneading device. The mixing order of each component is not particularly limited. Kneading conditions are not particularly limited, and the kneading is preferably carried out, for example, at 10°C to 50°C for 1 to 72 hours. After the kneading, it is preferable to bring the mixture into contact with the catalyst immediately before use.

In the acoustic wave probe according to the embodiment of the present invention, the liquid for forming the interlayer is applied onto the substrate and dried to form the interlayer, and the room temperature-curable silicone for forming the acoustic lens layer is laminated on the interlayer and cured, so that an acoustic wave probe in which the substrate and the acoustic lens layer are adhered to each other can be produced. The interlayer may be reacted separately from the acoustic lens layer, but it is preferable that the reaction proceeds and is completed at the same time in the step of curing the room temperature-curable silicone for forming the acoustic lens layer.

In a case where the other layers described above are further provided between the substrate and the interlayer, the other layers may be formed before the step of forming the interlayer on the substrate.

By curing the room temperature-curable silicone obtained in this manner, it is possible to obtain a silicone resin constituting the acoustic lens layer. Specifically, for example, a silicone resin can be obtained by heat-curing the composition for an acoustic lens at 20°C to 50°C for 5 to 500 minutes. A shape of the above-described silicone resin is not particularly limited. For example, the silicone resin may be formed into a preferred shape as an acoustic lens by a mold during the curing, or may be used as a desired acoustic lens by obtaining a sheet-like silicone resin and cutting the resin.

The acoustic wave probe according to the embodiment of the present invention can be preferably used for an acoustic wave measurement apparatus. The acoustic wave measurement apparatus according to the embodiment of the present invention is not limited to an ultrasound diagnostic apparatus or a photoacoustic wave measurement apparatus, but refers to a device that receives an acoustic wave reflected or generated by an object and displays the received acoustic wave as an image or a signal intensity.

In particular, the acoustic wave probe according to the embodiment of the present invention can be suitably used as an ultrasound probe equipped with a capacitive micromachined ultrasonic transducer (cMUT) as an ultrasonic transducer array, and it is preferable to use the acoustic wave probe according to the embodiment of the present invention in an ultrasound diagnostic apparatus including the ultrasound probe.

Specifically, the acoustic wave probe according to the embodiment of the present invention is preferably applied to an acoustic wave measurement apparatus such as the ultrasound diagnostic apparatus described in JP2003-169802A and the like, or the photoacoustic wave measurement apparatus described in JP2013-202050A, JP2013-188465A, and the like.

The configuration of the acoustic wave probe according to the embodiment of the present invention will be described in more detail below based on the configuration of the ultrasound probe in the ultrasound diagnostic apparatus described in Fig. 1. The ultrasound probe is a probe which particularly uses an ultrasonic wave as an acoustic wave in an acoustic wave probe. Therefore, a basic structure of the ultrasound probe can be applied to the acoustic wave probe as it is.

- Ultrasound probe -

An ultrasound probe 10 is a main component of the ultrasound diagnostic apparatus and has a function of generating an ultrasonic wave and transmitting and receiving an ultrasonic beam. As shown in Fig. 1, a configuration of the ultrasound probe 10 is provided in the order of an acoustic lens 1, an acoustic matching layer 2, a piezoelectric element layer 3, and a backing material 4 from a distal end portion (surface coming into contact with a living body which is a test object). In recent years, an ultrasound probe having a laminated structure in which an ultrasonic transducer (piezoelectric element) for transmission and an ultrasonic transducer (piezoelectric element) for reception are formed of materials different from each other has been proposed in order to receive high-order harmonics.

<Piezoelectric element layer>

The piezoelectric element layer 3 is a portion which generates an ultrasonic wave and in which an electrode is attached to both sides of a piezoelectric element. In a case where voltage is applied to the electrode, the piezoelectric element layer 3 generates an ultrasonic wave through repeated contraction and expansion of the piezoelectric element and through vibration.

A so-called ceramics inorganic piezoelectric body obtained by a polarization treatment of quartz crystals, single crystals such as $LiNbO_3$, $LiTaO_3$, and $KNbO_3$, thin films of ZnO and AlN, Pb(Zr,Ti)Os-based sintered body, and the like is widely used as the material constituting a piezoelectric element. In general, piezoelectric ceramics such as lead zirconate titanate (PZT) with good conversion efficiency are used.

In addition, sensitivity having a wider band width is required for a piezoelectric element detecting a reception wave on a high frequency side. For this reason, an organic piezoelectric body has been used in which an organic polymer material such as polyvinylidene fluoride (PVDF) is used as the piezoelectric element being suitable for a high frequency or a wide band.

Furthermore, cMUT using micro electro mechanical systems (MEMS) technology in which an array structure, which shows excellent short pulse characteristics, excellent wideband characteristics, and excellent mass productivity and has less characteristic variations, is obtained is described in JP2011-071842A or the like.

In the present invention, it is possible to preferably use any piezoelectric element material.

<Backing material>

The backing material 4 is provided on a rear surface of the piezoelectric element layer 3 and contributes to the improvement in distance resolution in an ultrasound diagnostic image by shortening the pulse width of an ultrasonic wave through the suppression of excess vibration.

<Acoustic matching layer>

The acoustic matching layer 2 is provided in order to reduce the difference in acoustic impedance between the piezoelectric element layer 3 and a test object and to efficiently transmit and receive an ultrasonic wave.

<Acoustic lens>

The acoustic lens 1 is provided to focus an ultrasonic wave in a slice direction by utilizing refraction to improve the resolution. In addition, it is necessary for the acoustic lens to achieve matching of an ultrasonic wave with acoustic impedance (1.4 to 1.7 Mrayl in a case of a human body) of a living body which is a test object after being closely attached to the living body.

That is, sensitivity of transmission and reception of an ultrasonic wave is improved using a material of which the acoustic velocity is sufficiently lower than that of a human body, and the acoustic impedance is close to a value of the skin of a human body, as the material of the acoustic lens 1.

<Interlayer>

One of the embodiments of the acoustic wave probe according to the embodiment of the present invention has a configuration in which the substrate in the acoustic wave probe according to the embodiment of the present invention corresponds to the acoustic matching layer 2, the above-described acoustic lens layer in the acoustic wave probe according to the embodiment of the present invention to the acoustic lens 1, and the acoustic matching layer 2 and the acoustic lens 1 are adhered to each other by the interlayer (not shown in Fig. 1) containing the above-described specific siloxane compound.

It is also preferable that the substrate in the acoustic wave probe according to the embodiment of the present invention is a constituent member other than the acoustic matching layer 2 in the periphery of the acoustic lens 1, in addition to the acoustic matching layer 2. In this case, the acoustic matching layer 2 and the constituent members other than the acoustic matching layer 2 in the periphery of the acoustic lens 1 are adhered to the acoustic lens 1 by the interlayer containing the above-described specific siloxane compound.

The operation of the ultrasound probe 10 having such a configuration will be described. The piezoelectric element layer 3 is resonated after applying a voltage to the electrodes provided on both sides of the piezoelectric element layer 3, and an ultrasonic signal is transmitted to a test object from the acoustic lens 1. During reception of the ultrasonic signal, the piezoelectric element layer 3 is vibrated using the signal (echo signal) reflected from the test object and this vibration is electrically converted into a signal to obtain an image.

In particular, it is possible to confirm for the acoustic lens layer in the acoustic wave probe according to the embodiment of the present invention to have a significant sensitivity improving effect at a transmission frequency of an ultrasonic wave of approximately 10 MHz or more as a general medical ultrasonic transducer. A particularly significant sensitivity improving effect can be expected particularly at a transmission frequency of an ultrasonic wave of 15 MHz or higher.

The acoustic lens layer in the acoustic wave probe according to the embodiment of the present invention can reduce the acoustic velocity to, for example, less than 900 m/s, and with an ultrasonic frequency of 10 MHz or more and less than 50 MHz, it is possible to obtain sufficiently accurate information about with regard to living tissues at a depth of 0.1 to 20 mm from a body surface.

Hereinafter, an apparatus in which the acoustic lens layer in the acoustic wave probe according to the embodiment of the present invention particularly functions to solve the problems of the related art will be described in detail.

The acoustic wave probe according to the embodiment of the present invention also exhibits excellent effects on apparatuses other than those described below.

- Ultrasound probe equipped with capacitive micromachined ultrasonic transducer (cMUT) -

In a case where the cMUT device described in JP2006-157320A, JP2011-71842A, or the like is used in an ultrasonic transducer array, its sensitivity is generally lower than that of a transducer using general piezoelectric ceramics (PZT).

However, as the acoustic lens layer used in the acoustic wave probe according to the embodiment of the present invention, as disclosed in WO2017/130890A, it is possible to compensate for the insufficient sensitivity of the cMUT by using an acoustic lens obtained from a specific composition for an acoustic wave probe. As a result, it is possible to bring the sensitivity of the cMUT closer to the performance of a transducer of the related art.

Since the cMUT device is produced by MEMS technology, it is possible to provide the market with an ultrasound probe having higher mass productivity and lower cost than a piezoelectric ceramic probe.

- Photoacoustic wave measurement apparatus using photoacoustic imaging -

Photoacoustic imaging (PAI) described in JP2013-158435A or the like displays an image or a signal intensity of an ultrasonic wave generated in a case where the inside of a human body is irradiated with light (an electromagnetic wave),

and the human tissue adiabatically expands due to the irradiated light.

- Ultrasonic endoscope -

Since the signal line cable of the ultrasonic endoscope described in JP2008-311700A or the like is longer than that of a transducer for the body surface due to its structure, it is a problem to improve the sensitivity of the transducer due to the cable loss of an ultrasonic wave. In addition, it is said that there is no effective method for improving sensitivity for this problem for the following reasons.

First, in a case where it is an ultrasound diagnostic apparatus for the body surface, an amplifier circuit, an analog-to-digital conversion integrated circuit (AD conversion IC), or the like can be installed at the tip of a transducer. On the other hand, since an ultrasonic endoscope is used by insertion thereof into the body, an installation space of a transducer is narrow, and it is difficult to install an amplifier circuit, an AD conversion IC, or the like at the tip of the transducer.

Secondly, a piezoelectric single crystal used in a transducer in an ultrasound diagnostic apparatus for the body surface is difficult to apply to a transducer with a transmission frequency of an ultrasonic wave of 10 to 15 MHz or higher due to its physical characteristics and process suitability. Meanwhile, since the ultrasound probe in the ultrasonic endoscope is generally a probe having a transmission frequency of an ultrasonic wave of 10 to 15 MHz or higher, it is difficult to improve the sensitivity by using a piezoelectric single crystal material.

However, as the acoustic lens layer used in the acoustic wave probe according to the embodiment of the present invention, as disclosed in WO2017/130890A, an acoustic lens obtained from a specific composition for an acoustic wave probe can be used for improving the sensitivity of the ultrasound transducer for an endoscope.

In addition, even in a case where the same transmission frequency of ultrasonic waves (for example, 15 MHz) is used, the effect is particularly exhibited in a case where the acoustic lens obtained from a specific composition for an acoustic lens is used as the acoustic lens layer used in the acoustic wave probe according to the embodiment of the present invention in the ultrasonic transducer for an endoscope, as disclosed in WO2017/130890A.

## Examples

Hereinafter, the present invention will be described in more detail with reference to Examples. The present invention is not limited to Examples below, except as specified in the present invention.

### <1. Substrate (B)>

Test pieces having a length of 80 mm, a width of 20 mm, and a thickness of 1 mm were cut out from various substrates (B-1) to (B-14) described in the note of the table below. One surface of each test piece of these substrates was treated with "UVO-Cleaner Model 42" (trade name, manufactured by Jelight Company Inc., central wavelength: 254 nm) for 2 minutes to prepare each of substrates (B-1) to (B-14) subjected to UV1 pretreatment.

One surface of a test piece of the substrate (B-4) was treated with a Xe excimer UV irradiation device "ASM86 Excimer" (trade name, manufactured by ASUMI GIKEN, Limited, central wavelength: 172 nm) to prepare a substrate (B-4) subjected to UV2 pretreatment.

One surface of a test piece of the substrate (B-4) was treated with a plasma treatment device "PM100" (trade name, manufactured by Yamato Scientific co., ltd.) to prepare a substrate (B-4) subjected to plasma pretreatment.

One surface of a test piece of the substrate (B-4) was treated with a corona discharge surface reforming device "CORONA MASTER PS-1M" (trade name, manufactured by Shinko Electric & Instrumentation Co., Ltd.) to prepare a substrate (B-4) subjected to corona pretreatment.

### <2. Preparation of liquid for interlayer>

#### (1) Preparation of liquid (m-1) for interlayer

2.4 g of hydrolyzed silicate "HAS-1" (trade name, manufactured by Colcoat Co,.Ltd., silica fraction: 21%) and 7.6 g of methanol were mixed to obtain a liquid (m-1) for an interlayer.

#### (2) Formation of liquid (m-2) for interlayer

2.4 g of hydrolysis silicate "HAS-1" (trade name, manufactured by Colcoat Co,.Ltd., silica fraction: 21%) and 0.3 g of N-2-(aminoethyl)-3-aminopropyl methyldimethoxysilane (trade name: KBM-603, manufactured by Shin-Etsu Chemical Co., Ltd.) were mixed with 7.3 g of methanol to obtain a liquid (m-2) for an interlayer.

(3) Formation of liquids (m-3) to (m-25) for interlayer

By performing a treatment of mixing each of the components shown in Table A below in the same manner as in (m-1) and (m-2) described above, liquids (m-3) to (m-25) for an interlayer were obtained.

In the liquids (m-18) to (m-20) for an interlayer, with regard to the hydrolyzable group included in the silicate compound, the components described in Table A were mixed and stirred until the hydrolysis rate described above reached 80% or more, the hydrolysis reaction was allowed to proceed, and then the liquid was subjected to a neutralization treatment with 0.02 N aqueous ammonia to have a pH of 5 to 7, thereby preparing the liquid for an interlayer.

[Table A]

| | | (m-1) | (m-2) | (m-3) | (m-4) | (m-5) | (m-6) | (m-7) | (m-8) | (m-9) | (m-10) |
|---|---|---|---|---|---|---|---|---|---|---|---|
| Raw material 1 | HAS-1 | 2.4 | 2.4 | 2.4 | 2.4 | 2.4 | 2.4 | 2.4 | 2.4 | 2.4 | 2.4 |
| Raw material 2 | Type | - | (SI-1) | (SI-2) | (SI-3) | (SI-4) | (TI-1) | (TI-2) | (TI-3) | (TI-4) | (AL-1) |
| | Amount | | 0.3 | 0.3 | 0.3 | 0.3 | 0.3 | 0.3 | 0.3 | 0.3 | 0.3 |
| Catalyst | 0.02 N Hydrochloric acid aqueous solution | - | - | - | - | - | - | - | - | - | - |
| Solvent | Methanol | 7.6 | 7.3 | 7.3 | 7.3 | 7.3 | 7.3 | 7.3 | 7.3 | 7.3 | 7.3 |
| | | (m-11) | (m-12) | (m-13) | (m-14) | (m-15) | (m-16) | (m-17) | | | |
| Raw material 1 | HAS-1 | 2.4 | 2.4 | 2.4 | 2.4 | 2.4 | 2.4 | 2.4 | | | |
| Raw material 2 | Type | (AL-2) | (AL-3) | (AL-4) | (ZR-1) | (ZR-2) | (ZR-3) | (ZR-4) | | | |
| | Amount | 0.3 | 0.3 | 0.3 | 0.3 | 0.3 | 0.3 | 0.3 | | | |
| Catalyst | 0.02 N Hydrochloric acid aqueous solution | - | - | - | - | - | - | - | | | |
| Solvent | Methanol | 7.3 | 7.3 | 7.3 | 7.3 | 7.3 | 7.3 | 7.3 | | | |
| | | (m-18) | (m-19) | (m-20) | (m-21) | (m-22) | (m-23) | (m-24) | (m-25) | | |
| Raw material 1 | HAS-1 | - | - | - | - | - | - | - | - | | |
| | Methyl silicate 51 | 1 | - | - | 1 | - | - | - | - | | |
| | MKC silicate MS56 | - | 0.9 | - | - | 0.9 | - | - | - | | |
| | TEOS | - | - | 1.7 | - | - | 1.7 | - | - | | |
| | TI-4 | - | - | - | - | - | - | 0.5 | - | | |
| | SI-4 | - | - | - | - | - | - | - | 0.5 | | |
| Raw material 2 | Type | - | - | - | - | - | - | - | - | | |
| | Amount | | | | | | | | | | |
| Catalyst | 0.02 N Hydrochloric acid aqueous solution | 0.7 | 0.7 | 0.7 | - | - | - | - | - | | |
| Solvent | Methanol | 8.3 | 8.4 | 7.6 | 9 | 9.1 | 8.3 | 9.5 | 9.5 | | |

(Note to table)

[Compound used for preparing liquid for interlayer]

In the following description, a silica fraction means a proportion of a hydrolyzed silicate compound or a silicate compound having a hydrolyzable group in a product on a mass basis.

In addition, a content of each component described in the tables is based on mass.

(Hydrolyzed silicate compound)

HAS-1: trade name, manufactured by Colcoat Co,.Ltd., silica fraction: 21%, weight-average molecular weight: 650

(Silicate compound having hydrolyzable group)

Methyl Silicate 51: trade name, manufactured by Colcoat Co,.Ltd., $Si_4O_3(OCH_3)_{10}$ (average tetramer), silica fraction: 51%, weight-average molecular weight: 470

MKC Silicate MS56: trade name, manufactured by Mitsubishi Chemical Corporation, $Si_mO_{m-1}(OCH_3)_{2m+2}$ (m = 2 to 100), silica fraction: 56%, weight-average molecular weight: 1,150

TEOS: tetraethoxysilane

(Silane coupling agent)

(SI-1): N-2-(aminoethyl)-3-aminopropylmethyldimethoxysilane (trade name: KBM-603, manufactured by Shin-Etsu Chemical Co., Ltd.)

(SI-2): 3-aminopropyltrimethoxysilane (trade name: KBM-903, manufactured by Shin-Etsu Chemical Co., Ltd.)

(SI-3): 3-trimethoxysilylpropyl succinic acid anhydride (trade name: X-12-967C, manufactured by Shin-Etsu Chemical Co., Ltd.)

(SI-4): 3-glycidoxypropyltrimethoxysilane (trade name: KBM-403, manufactured by Shin-Etsu Chemical Co., Ltd.)

(Titanium alkoxide compound)

(TI-1): isopropyl tri(N-aminoethyl-aminoethyl)titanate (trade name: PLENACT 44, manufactured by Ajinomoto Fine-Techno Co., Inc.)

(TI-2): diisopropyl bis(dioctylpyrophosphate) titanate (trade name: PLENACT 38S, manufactured by Ajinomoto Fine-Techno Co., Inc.)

(TI-3): isopropyltridecylbenzenesulfonyl titanate (trade name: PLENACT 9SA, manufactured by Ajinomoto Fine-Techno Co., Inc.)

(TI-4): tetra n-butyl titanate (trade name: ORGATIX TA-21, manufactured by Matsumoto Fine Chemical Co., Ltd.)

(Aluminum alkoxide compound)

(AL-1): aluminum trisacetylacetonate (trade name: ORGATIX AL-3100, manufactured by Matsumoto Fine Chemical Co., Ltd.)

(AL-2): aluminum bisethylacetoacetate monoacetylacetonate (trade name: ORGATIX AL-3200, manufactured by Matsumoto Fine Chemical Co., Ltd.)

(AL-3): aluminum trisethylacetoacetate (trade name: ORGATIX AL-3215, manufactured by Matsumoto Fine Chemical Co., Ltd.)

(AL-4): aluminum sec-butoxide (trade name: ASBD, manufactured by Kawaken Fine Chemicals Co.,Ltd.)

(Zirconium alkoxide compound)

(ZR-1): zirconium tetraacetylacetonate (trade name: ORGATIX ZC-150, manufactured by Matsumoto Fine Chemical Co., Ltd.)
(ZR-2): zirconium lactate ammonium salt (trade name: ORGATIX ZC-300, manufactured by Matsumoto Fine Chemical Co., Ltd.)
(ZR-3): zirconium tributoxy monoacetylacetonate (trade name: ORGATIX ZC-540, manufactured by Matsumoto Fine Chemical Co., Ltd.)
(ZR-4): zirconium tetra-n-butoxide (trade name: ORGATIX ZA-65, manufactured by Matsumoto Fine Chemical Co., Ltd.)

<3. Preparation of resin for acoustic lens>

(1) Preparation of resin (L-1) for acoustic lens

54.0 parts by mass of a vinyl-terminated diphenylsiloxane-dimethylsiloxane copolymer ("PDV-0535" manufactured by Gelest, Inc., mass-average molecular weight: 47,500, diphenylsiloxane content: 5 mol%), 1.0 part by mass of polymethylhydrosiloxane ("HMS-991" manufactured by Gelest, Inc., mass-average molecular weight: 1,600), and 45.0 parts by mass of true sphere-shaped surface-treated silica ("QSG-30" manufactured by Shin-Etsu Chemical Co., Ltd., average primary particle diameter: 30 nm, surface-treated product with methyltrimethoxysilane and hexamethyldisilazane (HMDS), methanol hydrophobicity degree: 67% by mass) were kneaded with a kneader for 2 hours at a set temperature of 23°C to obtain a uniform paste.

0.05 parts by mass of a platinum catalyst solution (manufactured by Gelest, Inc., trade name "SIP6830.3", Pt concentration: 3% by mass) was added thereto, and the mixture was degassed under reduced pressure to prepare an uncured resin (L-1) for an acoustic lens.

(2) Preparation of resin (L-2) for acoustic lens

An uncured resin (L-2) for an acoustic lens was prepared in the same manner as in the preparation of the resin (L-1) for an acoustic lens, except that the vinyl-terminated diphenylsiloxane-dimethylsiloxane copolymer was changed to 54.0 parts by mass of "PDV-0341" (manufactured by Gelest, Inc., mass-average molecular weight: 62,000, diphenylsiloxane content: 3 mol%).

(3) Preparation of resin (L-3) for acoustic lens

An uncured resin (L-3) for an acoustic lens was prepared in the same manner as in the preparation of the resin (L-1) for an acoustic lens, except that the silica was changed to 45.0 parts by mass of surface-treated titanium oxide "TITONE R-24" (trade name, manufactured by SAKAI CHEMICAL INDUSTRY CO.,LTD., average primary particle diameter: 0.20 $\mu$m, surface treatment agent: silica-alumina-organic).

(4) Preparation of resin (L-4) for acoustic lens

An uncured resin (L-4) for an acoustic lens was prepared in the same manner as in the preparation of the resin (L-1) for an acoustic lens, except that the silica was changed to 45.0 parts by mass of non-spherical surface-treated fumed silica ("AEROSIL (registered trademark) NAX50" manufactured by Nippon Aerosil Co., Ltd., average primary particle diameter: 30 nm, surface treated product with hexamethyldisilazane (HMDS), methanol hydrophobicity degree: 28% by mass).

(5) Preparation of resin (L-5) for acoustic lens

An uncured resin (L-5) for an acoustic lens was prepared in the same manner as in the preparation of the resin (L-1) for an acoustic lens, except that the vinyl-terminated diphenylsiloxane-dimethylsiloxane copolymer was changed to 54.0 parts by mass of vinyl-terminated dimethylsiloxane copolymer "DMS-V41" (manufactured by Gelest, Inc., mass-average molecular weight: 62,700, diphenylsiloxane content: 0 mol%).

<4. Production of laminate>

The entire surface of the above-described substrate (B-1) subjected to the UV1 pretreatment was coated with the above-described liquid (m-1) for an interlayer using a cotton swab, and dried at 50°C for 15 minutes to form an interlayer (M-1).

The substrate to which the interlayer was applied was set in a mold, the resin (L-1) for an acoustic lens, which had been mixed and defoamed in advance, was poured onto the treatment surface, the resin for a lens was placed such that the thickness of the resin for a lens was 1 mm (the total thickness of the laminate was approximately 2 mm), and then the resin was cured at 100°C for 10 minutes. As a result, a laminate No. 101 in which an acoustic lens layer (L) was applied to a substrate (B) through an interlayer (M) was obtained.

Laminates Nos. 102 to 140 and c01 to c07 were produced in the same manner as in the production of the laminate No. 101 described above, except that the substrate (B), the interlayer (M), and the acoustic lens layer (L) were changed to configurations shown in Tables 1-1 and 1-2.

The laminates Nos. 101 to 140 are the acoustic wave probes according to the embodiment of the present invention, and the laminates Nos. c01 to c07 are acoustic wave probes for comparison.

An average layer thickness of the interlayer (M) was calculated as follows.

The laminate produced above was randomly cut at five positions, and the cross section of each interlayer (M) was observed with a scanning electron microscope (S-5500 (trade name), manufactured by Hitachi High-Tech Corporation) at 50,000 times to obtain a thickness of the interlayer (M) at each cross section. An average value of the obtained five thickness values was defined as the average layer thickness. The average layer thickness is described in the column of film thickness in the tables below.

<5. Evaluation of laminate>

The peel strength, heat cycle durability, and hydrogen peroxide plasma sterilization resistance of the laminate produced above were evaluated as follows. The obtained results are summarized in Tables 1-1 and 1-2. The hydrogen peroxide plasma sterilization resistance is described as $H_2O_2$ gas sterilization durability in the tables.

[Test Example 1] Evaluation of peel strength

A test piece having a length of 80 mm and a width of 10 mm (a thickness of about 2 mm) was cut out from the laminate produced above, thereby obtaining a laminated test piece. A notch having a width of 1 cm was formed in the interlayer on a side of the laminated test piece having a width of 10 mm. For the laminated test piece with the cut, a 90° peel strength between the substrate (B) and the acoustic lens layer (L) was measured at 25°C by gripping the end of the 1 cm wide cut on the acoustic lens layer side and peeling off the cut by 20 mm at a peeling rate of 2 mm/min along a longitudinal direction while maintaining an angle between the substrate and the acoustic lens layer at 90°. The peel strength was a value measured with a force gauge, and the unit thereof was N/cm. The average value of the measured entire region of the peel strength was evaluated according to the following standard.

<Evaluation standard for peel strength>

AA: 12 N/cm or more, or cohesive destruction of the resin constituting the acoustic lens layer occurred.
A: 10 N/cm or more and less than 12 N/cm
B: 6 N/cm or more and less than 10 N/cm
C: 2 N/cm or more and less than 6 N/cm
D: less than 2 N/cm

In a case where the peel strength was less than 12 N/cm, cohesive destruction of the resin constituting the acoustic lens layer did not occur.

[Test Example 2] Evaluation of heat cycle durability

The laminated test piece produced in Test Example 1 described above was subjected to a heat cycle test in which one cycle was set as 2 hours at -20°C and 2 hours at 60°C using a constant temperature and humidity machine

(manufactured by SATUKI CHEMICAL MACHINE WORKS, LTD., KHWV-40HP (trade name)) for 500 cycles. The 90° peel strength of the laminated test piece after 500 cycles of the heat cycle test was measured according to the method described in Test Example 1 above.

The 90° peel strength before the heat cycle treatment measured in Test Example 1 above was defined as "PSB (1)", the 90° peel strength after the heat cycle treatment was defined as "PSA (1)", and a proportion of "PSA (1)" to "PSB (1)" {(PSA (1)/PSB (1)) × 100 (%)} was determined and evaluated according to the following standard.

<Evaluation standard for heat cycle resistance>

AA: 80% or more
A: 60% or more and less than 80%
B: 40% or more and less than 60%
C: 20% or more and less than 40%
D: less than 20%

[Test Example 3] Evaluation of hydrogen peroxide plasma sterilization resistance

The laminated test piece produced in Test Example 1 was subjected to a low-temperature plasma sterilization treatment 50 times in an advanced course of a hydrogen peroxide gas sterilizer ("STERRAD (registered trademark) NX" manufactured by ASP). The 90° peel strength of the laminated test piece after 50 times of the hydrogen peroxide plasma sterilization test was measured according to the method described in Test Example 1 above. The laminated test piece was placed on a stainless steel plate such that the acoustic lens layer (L) was on the upper side and the substrate (B) was on the lower side; and the above-described hydrogen peroxide plasma sterilization treatment was performed by decomposing hydrogen peroxide gas and stronger hydrogen peroxide gas generated by plasma irradiation and allowing the activated substance to hardly permeate.

The 90° peel strength before the hydrogen peroxide plasma sterilization treatment measured in Test Example 1 above was defined as "PSB (2)", the 90° peel strength after the hydrogen peroxide plasma sterilization test was defined as "PSA (2)", and a proportion of "PSA (2)" to "PSB (2)" {(PSA (2)/PSB (2)) × 100 (%)} was obtained and evaluated according to the following standard.

<Evaluation standard for hydrogen peroxide plasma sterilization resistance>

AA: 80% or more
A: 60% or more and less than 80%
B: 40% or more and less than 60%
C: 20% or more and less than 40%
D: less than 20%

[Table 1-1]

| No. | | 101 | 102 | 103 | 104 | 105 | 106 | 107 | 108 | 109 | 110 | 111 | 112 | 113 |
|---|---|---|---|---|---|---|---|---|---|---|---|---|---|---|
| Substrate (B) | Material | (B-1) acrylic | (B-2) PC | (B-3) GPPS | (B-4) crosslinked PS | (B-5) epoxy | (B-6) phenol | (B-7) UPes | (B-8) PI | (B-9) melamine | (B-10) glass | (B-11) ceramics | (B-12) graphite | (B-13) Al |
| | Pretreatment | UV1 | UV1 | UV1 | UV1 | UV1 | UV1 | UV1 | UV1 | UV1 | UV1 | UV1 | UV1 | UV1 |
| Interlayer (M) | Material | (M-1) | (M-1) | (M-1) | (M-1) | (M-1) | (M-1) | (M-1) | (M-1) | (M-1) | (M-1) | (M-1) | (M-1) | (M-1) |
| | Film thickness | 440 nm | 420 nm | 410 nm | 430 nm | 400 nm | 420 nm | 430 nm | 440 nm | 420 nm | 410 nm | 410 nm | 420 nm | 440 nm |
| Acoustic lens layer (L) | | (L-1) | (L-1) | (L-1) | (L-1) | (L-1) | (L-1) | (L-1) | (L-1) | (L-1) | (L-1) | (L-1) | (L-1) | (L-1) |
| Peel strength | | A | A | A | A | A | A | A | A | A | A | A | A | A |
| Heat cycle resistance | | B | B | B | A | A | A | A | A | A | B | B | B | C |
| $H_2O_2$ gas sterilization dur-ability | | A | B | B | A | C | B | A | A | C | B | B | B | C |

| No. | | 114 | 115 | 116 | 117 | 118 | 119 | 120 | 121 | 122 | 123 | 124 | 125 | 126 |
|---|---|---|---|---|---|---|---|---|---|---|---|---|---|---|
| Substrate (B) | Material | (B-14) Cu | (B-4) | (B-4) | (B-4) | (B-4) | (B-4) | (B-4) | (B-4) | (B-4) | (B-4) | (B-4) | (B-4) | (B-4) |
| | Pretreatment | UV1 | UV1 | UV1 | UV1 | UV1 | UV1 | UV1 | UV1 | UV1 | UV1 | UV1 | UV1 | UV1 |
| Interlayer (M) | Material | (M-1) | (M-2) | (M-3) | (M-4) | (M-5) | (M-6) | (M-7) | (M-8) | (M-9) | (M-10) | (M-11) | (M-12) | (M-13) |
| | Film thickness | 420 nm | 560 nm | 530 nm | 510 nm | 600 nm | 580 nm | 550 nm | 570 nm | 570 nm | 630 nm | 620 nm | 660 nm | 690 nm |
| Acoustic lens layer (L) | | (L-1) | (L-1) | (L-1) | (L-1) | (L-1) | (L-1) | (L-1) | (L-1) | (L-1) | (L-1) | (L-1) | (L-1) | (L-1) |
| Peel strength | | A | AA | AA | A | A | A | A | A | AA | AA | AA | AA | AA |
| Heat cycle resistance | | C | A | A | AA | A | AA | AA | AA | AA | AA | AA | AA | AA |
| $H_2O_2$ gas sterilization dur-ability | | C | A | A | A | AA | A | A | A | A | AA | AA | AA | AA |

[Table 1-2]

| No. | | 127 | 128 | 129 | 130 | 131 | 132 | 133 | 134 | 135 | 136 | 137 | 138 |
|---|---|---|---|---|---|---|---|---|---|---|---|---|---|
| Substrate (B) | Material | (B-4) | (B-4) | (B-4) | (B-4) | (B-4) | (B-4) | (B-4) | (B-4) | (B-4) | (B-4) | (B-4) | (B-4) |
| | Pretreatment | UV1 | UV1 | UV1 | UV1 | UV1 | UV1 | UV1 | UV2 | Plasma | Corona | - | UV1 |
| Interlayer (M) | Material | (M-14) | (M-15) | (M-16) | (M-17) | (M-18) | (M-19) | (M-20) | (M-1) | (M-1) | (M-1) | (M-1) | (M-1) |
| | Film thickness | 780 nm | 880 nm | 740 nm | 800 nm | 40 nm | 80 nm | 40 nm | 430 nm | 410 nm | 400 nm | 420 nm | 410 nm |
| Acoustic lens layer (L) | | (L-1) | (L-1) | (L-1) | (L-1) | (L-1) | (L-1) | (L-1) | (L-1) | (L-1) | (L-1) | (L-1) | (L-2) |
| Peel strength | | A | A | A | AA | A | A | A | A | A | A | B | A |
| Heat cycle resistance | | A | A | A | A | A | B | B | A | A | B | C | B |
| $H_2O_2$ gas sterilization durability | | AA | AA | AA | AA | B | A | B | A | A | B | C | C |
| No. | | 139 | 140 | c01 | c02 | c03 | c04 | c05 | c06 | c07 | | | |
| Substrate (B) | Material | (B-4) | (B-4) | (B-4) | (B-4) | (B-4) | (B-4) | (B-4) | (B-4) | (B-4) | | | |
| | Pretreatment | UV1 | UV1 | UV1 | UV1 | UV1 | UV1 | UV1 | UV1 | UV1 | | | |
| Interlayer (M) | Material | (M-1) | (M-1) | - | (M-21) | (M-22) | (M-23) | (M-24) | (M-25) | (M-1) | | | |
| | Film thickness | 410 nm | 430 nm | | 15 nm | 20 nm | 10 nm | 6.1 μm | 1.2 μm | 430 nm | | | |
| Acoustic lens layer (L) | | (L-3) | (L-4) | (L-1) | (L-1) | (L-1) | (L-1) | (L-1) | (L-1) | (L-5) | | | |
| Peel strength | | B | A | D | A | A | A | C | B | A | | | |
| Heat cycle resistance | | AA | C | D | C | D | C | D | D | D | | | |
| $H_2O_2$ gas sterilization durability | | A | C | D | D | D | D | D | D | D | | | |

<Note to table>

[Substrate (B)]

(Material)

(B-1): acrylic resin (trade name: ACRYLITE L, manufactured by Mitsubishi Chemical Corporation)
(B-2): polycarbonate (PC) resin (trade name: PANLITE L-1225Y, manufactured by Teijin Limited)
(B-3): general-purpose polystyrene (GPPS) resin (trade name: PSJ-polystyrene HF77, manufactured by PS Japan Co., Ltd.)
(B-4): crosslinked polystyrene (crosslinked PS) resin (trade name: REXOLITE, manufactured by Ensinger)
(B-5): product obtained by curing an epoxy resin (trade name: two-liquid epoxy resin-based adhesive 6100, manufactured by ARTECO S.R.L.) at 25°C for 24 hours
(B-6): product obtained by heating and curing a phenol resin (trade name: SUMICON PM-760, manufactured by Sumitomo Bakelite Co., Ltd.) at 180°C for 30 minutes
(B-7): product obtained by heating and curing an unsaturated polyester (UPes) resin (trade name: YUPIKA 4072, manufactured by Nippon YUPIKA Co., Ltd.) at 100°C for 24 hours
(B-8): polyimide (PI) resin (thermosetting polyimide resin, trade name: KAPTON, manufactured by Du Pont-Toray Co., Ltd.)
(B-9): product obtained by heating and curing a melamine resin (trade name: MURAS MBF-C, manufactured by Panasonic Corporation) at 150°C for 10 minutes
(B-10): glass
(B-11): ceramics
(B-12): graphite
(B-13): aluminum (Al)
(B-14): copper (Cu)

(Pretreatment)

UV1, UV2, plasma, and corona: each means the above-described UV1 pretreatment, UV2 pretreatment, plasma pretreatment, and corona pretreatment.

"-" indicates that the pretreatment had not been performed.

[Interlayer (M)]

Interlayers (M-1) to (M-25): each was produced using the above-described liquid (m-1) to (m-25) for an interlayer.

[Acoustic lens layer (L)]

Acoustic lens layers (L-1) to (L-5): each was produced using the above-described resins (L-1) to (L-5) for an acoustic lens

From Tables 1-1 and 1-2, the following was found.

The laminate No. c01 for comparison did not satisfy the definition of the present invention in that the substrate and the acoustic lens layer defined in the present invention were directly bonded to each other without an interlayer. In the laminate No. c01 for comparison, the initial peel strength was low, the initial adhesiveness was deteriorated, and the hydrogen peroxide plasma sterilization resistance was deteriorated.
In the laminates Nos. c02 to c06 for comparison, the interlayer was interposed between the substrate and the acoustic lens layer defined in the present invention, but the interlayer did not contain the siloxane compound derived from a hydrolyzed silicate compound, which did not satisfy the definition of the present invention. All of the laminates Nos. c02 to c06 for comparison were deteriorated in hydrogen peroxide plasma sterilization resistance.
The laminate No. c07 for comparison did not satisfy the definition of the present invention in that, although the interlayer defined in the present invention was interposed between the substrate and the acoustic lens layer, the acoustic lens layer was a layer obtained by curing room temperature-curable silicone which did not contain the constitutional unit

having a phenyl group. The laminate No. c07 for comparison was deteriorated in hydrogen peroxide plasma sterilization resistance.

On the other hand, it was found that in all of the laminates Nos. 101 to 140 in which the substrate, the interlayer, and the acoustic lens layer, which are defined in the present invention, were laminated in this order, the initial peel strength was high, and the peel strength after the hydrogen peroxide plasma sterilization was also maintained at 20% or more of the initial value, and thus the initial adhesiveness and the hydrogen peroxide plasma sterilization resistance were excellent.

The present invention has been described with the embodiments thereof, any details of the description of the present invention are not limited unless described otherwise, and it is obvious that the present invention is widely construed without departing from the gist and scope of the present invention described in the accompanying claims.

The present application claims the priority of JP2022-104704 filed in Japan on June 29, 2022, the contents of which are incorporated herein by reference, as a part of the description of the present specification.

Explanation of References

1: acoustic lens
2: acoustic matching layer
3: piezoelectric element layer
4: backing material
7: housing
9: cord
10: ultrasound probe

## Claims

1. An acoustic wave probe comprising:

    a substrate;
    an interlayer on the substrate; and
    an acoustic lens layer on the interlayer,
    wherein the interlayer is a layer containing a siloxane compound,
    the siloxane compound has a constitutional component derived from a hydrolyzed silicate compound, and
    the acoustic lens layer is a layer obtained by curing room temperature-curable silicone, in which the room temperature-curable silicone includes a constitutional unit having a phenyl group.

2. The acoustic wave probe according to claim 1,
wherein the substrate contains at least one of iron, a nonferrous metal, an inorganic material other than metal, or an organic material.

3. The acoustic wave probe according to claim 2,
wherein the nonferrous metal includes at least one of aluminum, titanium, magnesium, nickel, copper, lead, zinc, tin, chromium, tungsten, cobalt, or an alloy of at least two of these metals.

4. The acoustic wave probe according to claim 2 or 3,
wherein the inorganic material other than metal includes at least one of glass, ceramics, or graphite.

5. The acoustic wave probe according to any one of claims 2 to 4,
wherein the organic material includes at least one of a thermoplastic resin or a thermosetting resin.

6. The acoustic wave probe according to claim 5,
wherein the thermoplastic resin includes at least one of an acrylic resin, a polycarbonate resin, a styrene-based resin, or a polyphenylene sulfide resin.

7. The acoustic wave probe according to claim 5 or 6,
wherein the thermoplastic resin is a crosslinked thermoplastic resin.

**8.** The acoustic wave probe according to any one of claims 1 to 7, wherein the substrate is a substrate in which a surface on a side of the interlayer has been subjected to a hydrophilization treatment.

**9.** The acoustic wave probe according to claim 8, wherein the substrate is a substrate in which the surface on the side of the interlayer has been subjected to at least one treatment of an ultraviolet treatment, an ozone treatment, a plasma treatment, or a corona treatment.

**10.** The acoustic wave probe according to any one of claims 1 to 9, wherein the interlayer includes a constitutional component derived from at least one of a silane coupling agent, a titanium alkoxide compound, an aluminum alkoxide compound, or a zirconium alkoxide compound.

**11.** The acoustic wave probe according to any one of claims 1 to 10, wherein the room temperature-curable silicone is a silicone composition containing vinyl silicone and hydrosilicone.

**12.** The acoustic wave probe according to claim 11, wherein the silicone composition contains surface-treated oxide particles.

**13.** The acoustic wave probe according to claim 12, wherein the silicone composition contains surface-treated spherical silica particles.

**14.** The acoustic wave probe according to any one of claims 1 to 13, wherein the acoustic wave probe is an ultrasound probe.

**15.** An acoustic wave measurement apparatus comprising: the acoustic wave probe according to any one of claims 1 to 13.

**16.** An ultrasound diagnostic apparatus comprising: the acoustic wave probe according to claim 14.

FIG. 1

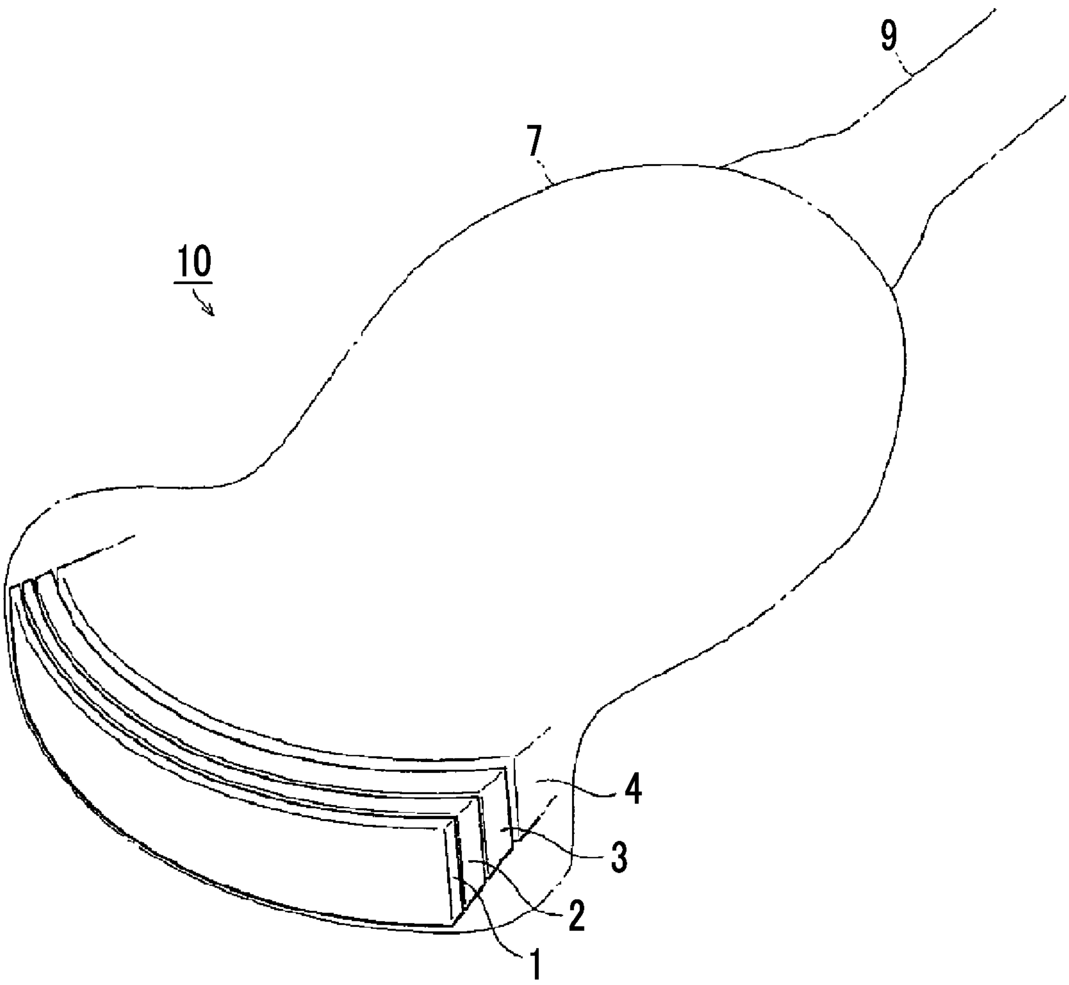

## INTERNATIONAL SEARCH REPORT

International application No.
**PCT/JP2023/023757**

### A. CLASSIFICATION OF SUBJECT MATTER

***A61B 8/00***(2006.01)i; ***H04R 17/00***(2006.01)i
FI: A61B8/00; H04R17/00 330J

According to International Patent Classification (IPC) or to both national classification and IPC

### B. FIELDS SEARCHED

Minimum documentation searched (classification system followed by classification symbols)

A61B8/00-8/15; H04R17/00-17/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | WO 2017/130890 A1 (FUJIFILM CORP.) 03 August 2017 (2017-08-03) paragraphs [0015]-[0017], [0021], [0060], [0061], [0089], [0092], [0107] | 1-16 |
| Y | JP 2001-40286 A (GENERAL ELECTRIC CO.) 13 February 2001 (2001-02-13) paragraphs [0001], [0005], [0022], [0023] | 1-16 |
| Y | WO 2020/223182 A1 (DOW SILICONES CORP.) 05 November 2020 (2020-11-05) paragraphs [0008]-[0010] | 1-16 |
| Y | JP 2018-4901 A (FUJI XEROX CO., LTD.) 11 January 2018 (2018-01-11) paragraph [0020] | 1-16 |
| Y | JP 2016-25612 A (KONICA MINOLTA, INC.) 08 February 2016 (2016-02-08) paragraph [0036] | 2-10 |
| Y | JP 2021-142109 A (CANON MEDICAL SYSTEMS CORP.) 24 September 2021 (2021-09-24) paragraph [0046] | 8-10 |
| Y | WO 2021/065990 A1 (DOW TORAY CO., LTD.) 08 April 2021 (2021-04-08) paragraphs [0047]-[0049] | 10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 August 2023** | **29 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
**PCT/JP2023/023757**

**C. DOCUMENTS CONSIDERED TO BE RELEVANT**

| Category* | Citation of document, with indication, where appropriate, of the relevant passages | Relevant to claim No. |
|---|---|---|
| A | JP 2016-040798 A (KONICA MINOLTA, INC.) 24 March 2016 (2016-03-24)<br>entire text, all drawings | 1-16 |
| A | WO 2021/044823 A1 (FUJIFILM CORP.) 11 March 2021 (2021-03-11)<br>entire text, all drawings | 1-16 |

Form PCT/ISA/210 (second sheet) (January 2015)

**INTERNATIONAL SEARCH REPORT**
**Information on patent family members**

International application No.
**PCT/JP2023/023757**

| Patent document cited in search report | Publication date (day/month/year) | Patent family member(s) | Publication date (day/month/year) |
|---|---|---|---|
| WO 2017/130890 A1 | 03 August 2017 | US 2018/0344287 A1<br>paragraphs [0044]-[0054], [0062], [0144]-[0146], [0173], [0177], [0214]-[0125]<br>EP 3409209 A4 | |
| JP 2001-40286 A | 13 February 2001 | EP 1063270 A2<br>paragraphs [0001], [0005], [0023], [0024]<br>US 6231990 B2 | |
| WO 2020/223182 A1 | 05 November 2020 | JP 2022-530753 A<br>US 2022/0204770 A1<br>EP 3963018 A1<br>CN 113677769 A<br>KR 10-2022-0002477 A | |
| JP 2018-4901 A | 11 January 2018 | CN 107561894 A | |
| JP 2016-25612 A | 08 February 2016 | (Family: none) | |
| JP 2021-142109 A | 24 September 2021 | US 2021/0321984 A1<br>paragraph [0052] | |
| WO 2021/065990 A1 | 08 April 2021 | US 2022/0325137 A1<br>paragraphs [0060], [0061]<br>EP 4039377 A1<br>CN 114430758 A<br>KR 10-2022-0083717 A | |
| JP 2016-040798 A | 24 March 2016 | (Family: none) | |
| WO 2021/044823 A1 | 11 March 2021 | US 2022/0172701 A1<br>entire text, all drawings<br>EP 4026872 A1<br>CN 114269860 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017130890 A
- JP 2005103294 A
- JP 2007099582 A
- JP 2014114175 A
- JP 2003169802 A
- JP 2013202050 A
- JP 2013188465 A
- JP 2011071842 A
- JP 2006157320 A
- JP 2013158435 A
- JP 2008311700 A
- JP 2022104704 A


### Non-patent literature cited in the description


- Chemical Engineering Handbook. Maruzen Co., Ltd.